(19) Europäisches Patentamt / European Patent Office / Office européen des brevets

(11) **EP 4 446 412 A1**

(12) **EUROPEAN PATENT APPLICATION**
published in accordance with Art. 153(4) EPC

(43) Date of publication:
**16.10.2024 Bulletin 2024/42**

(21) Application number: **22904246.0**

(22) Date of filing: **07.12.2022**

(51) International Patent Classification (IPC):
**C12N 11/04** (2006.01)  **C12Q 1/02** (2006.01)
**G01N 33/48** (2006.01)

(52) Cooperative Patent Classification (CPC):
**C12N 11/04; C12Q 1/02; G01N 33/48**

(86) International application number:
**PCT/JP2022/045039**

(87) International publication number:
**WO 2023/106318 (15.06.2023 Gazette 2023/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.12.2021 JP 2021199634**

(71) Applicant: **National Institutes for Quantum
Science and
Technology
Chiba 263-8555 (JP)**

(72) Inventor: **SUZUKI, Michiyo
Chiba-shi, Chiba 263-8555 (JP)**

(74) Representative: **Graf von Stosch
Patentanwaltsgesellschaft mbH
Prinzregentenstraße 22
80538 München (DE)**

(54) **NEMATODE-SEALED CAPSULE, METHOD FOR PRODUCING NEMATODE-SEALED CAPSULE, AND USE OF NEMATODE-SEALED CAPSULE**

(57)   A nematode-encapsulated capsule of the present disclosure includes: an encapsulation composition containing a nematode; and at least one layer made of membrane with which the encapsulation composition is encapsulated. The membrane contains, as a main component, a water-soluble polymer that gelatinizes by reacting with a cation.

FIG. 1

NEMATODE (ADULT)

CAPSULE

1 mm

EP 4 446 412 A1

**Description**

Technical Field

The present invention relates to a capsule in which a nematode is encapsulated (which, hereinafter, may be referred to as "nematode-encapsulated capsule"), a method for producing the nematode-encapsulated capsule, and an application of the nematode-encapsulated capsule. Specifically, the present invention relates to a nematode-encapsulated capsule, a method for producing the nematode-encapsulated capsule, a method for evaluating response of nematodes, a cancer test method, a nematode response evaluation kit, and a production kit for producing the nematode-encapsulated capsule.

Background Technology

As disclosed in Patent Literatures 1 and 2, cancer tests using nematodes in which urine is used as a test sample have recently been drawing attention. Such an attention has led to increased needs for culture of nematodes by medical institutes or individuals. For example, as disclosed in Non-Patent Literature 1, for supply of nematodes between trained people such as researchers of nematodes, nematodes are transported at normal temperature in a state in which the nematodes are cultured on an agar medium to which *Escherichia coli* that will be food for the nematodes is applied. Then, the researchers and/or the like collect the nematodes from the agar medium and culture the nematodes under conditions suitable for an intended assay.

Further, Non-Patent Literature 2 discloses a method of an experiment using nematodes, with use of a dedicated pack in which nematodes and food are encapsulated. This experimental method is a method developed for space experiments.

Citation List

[Patent Literature]

[Patent Literature 1]
Japanese Patent Application Publication Tokukai No. 2018-61515
[Patent Literature 2]
International Publication No. WO 2020/218501

[Non-patent Literature]

[Non-patent Literature 1]
Stiernagle, T. Maintenance of C. elegans (February 11, 2006), WormBook, ed. The C. elegans Research Community, WormBook

[Non-patent Literature 2]
Akira Higashibata et al., Microgravity elicits reproducible alterations in cytoskeletal and metabolic gene and protein expression in space-flown Caenorhabditis elegans. npj Microgravity (2016) 2,15022.

Summary of Invention

Technical Problem

For a method of supplying nematodes disclosed in Non-Patent Literature 1, suitable knowledge, technology and facilities are required, and it is not possible for everyone to easily use the method. Further, the experimental method disclosed in Non-Patent Literature 2 employs a unique culture condition etc., and thus the method cannot be always suitable for a cancer test using nematodes and various basic biological experiments. In light of the above, desired is development of a technique for allowing nematodes to be easily used for experiments, assays, etc. by a person who is not a so-called trained person, such as a researcher who, for example, routinely conducts an experiment etc. using nematodes and is familiar with culture of nematodes or an experiment of nematodes.

An object of an aspect of the present invention is to realize a technology for allowing even an untrained person to easily use a nematode(s).

Means to Solve the Problem

As a result of diligent studies, the inventor of the present invention has found that using a capsule obtained by encapsulating nematodes in a biocompatible artificial membrane allows even an untrained person to easily use the nematodes. As a result, the inventor has accomplished the present invention.

A nematode-encapsulated capsule according to an aspect of the present invention includes: an encapsulation composition containing a nematode; and at least one layer made of membrane with which the encapsulation composition is encapsulated, the membrane containing, as a main component, a water-soluble polymer that gelatinizes by reacting with a cation.

Advantageous Effects of Invention

An aspect of the present invention can allow even an untrained person to easily use a nematode(s).

Brief Description of Drawings

Fig. 1 is a microscopic image obtained by capturing from above an image of a spherical capsule in which adult nematodes were encapsulated, in Example 1.
Fig. 2 is a microscopic image obtained by capturing from above an image of spherical capsules which had various sizes and in each of which adult nematodes were encapsulated, in Example 1.
Fig. 3 is a microscopic image obtained by capturing from above an image of spherical capsules which had arbitrary shapes and in each of which adult nematodes were encapsulated, in Example 1.
Fig. 4 is a microscopic image obtained by capturing from above an image of a spherical capsule in which nematode eggs were encapsulated, in Example 2.
Fig. 5 is a microscopic image obtained by capturing, from above every day, an image of a state of a spherical capsule in which nematode eggs were encapsulated, in Example 3.
Fig. 6 is a microscopic image obtained by capturing, from above every day, an image of a state of a spherical capsule in which adult nematodes were encapsulated, in Example 4.
Fig. 7 is a microscopic image obtained by capturing from above an image of a state in which adult nematodes and nematode larvae were crawling out of a nematode-encapsulated capsule, in Example 4.
Fig. 8 is a microscopic image obtained by capturing, from above every day, an image of a state of a spherical capsule in which adult nematodes having been exposed to radiation were encapsulated, in Example 5.
Fig. 9 is a microscopic image obtained by capturing from above an image of a state in which adult nematodes having been exposed to radiation were crawling out of the nematode-encapsulated capsule, in Example 5.
Fig. 10 is a microscopic image obtained by capturing, from above every day, an image of a state of a spherical capsule in which adult nematodes having been exposed to a hatching inhibitor were encapsulated, in Example 6.
Fig. 11 is a microscopic image obtained by capturing from above an image of a state in which adult nematodes having been exposed to the hatching inhibitor were crawling out of the nematode-encapsulated capsule, in Example 6.
Fig. 12 is a microscopic image obtained by capturing, from above every day, an image of a state of a spherical capsule where adult nematodes having been cultured under a condition in which no hatching inhibitor had been added were encapsulated, in Example 6.
Fig. 13 is a view showing a state in which spherical capsules of two different sizes in each of which adult nematodes were encapsulated were sealed in plastic dishes, in Example 7.
Fig. 14 is an image obtained by capturing, from above every day, an image of spherical capsules in which adult nematodes were encapsulated scattered on leaf mold that had been spread on a plastic dish, in Example 8.
Fig. 15 is a view showing a result obtained when a capsule in which adult nematodes were encapsulated was supplied on a nematode assay plate A1 and a supply part was observed over time, in Example 9.
Fig. 16 is a view showing a result obtained when a capsule in which adult nematodes were encapsulated was supplied on a nematode assay plate A2 and a supply part was observed over time, in Example 9.
Fig. 17 is a view showing a result obtained when nematodes were supplied on a nematode assay plate A1 by a conventional method and a state of a supply part was observed over time, in Comparative Example 1.
Fig. 18 is a view showing a result obtained when nematodes were supplied on a nematode assay plate A2 and a state of a supply part was observed over time, in Comparative Example 1.

Fig. 19 is a view showing a result of a chemotaxis assay in which a capsule where adult nematodes were encapsulated were supplied on a nematode assay plate A3 and in which chemotaxis of the nematodes with respect to a volatile substance was tested, in Example 10.

Fig. 20 is a view showing a result of a chemotaxis assay in which nematodes were supplied by a conventional method on a nematode assay plate A3 and chemotaxis of nematodes with respect to a volatile substance was tested, in Comparative Example 2.

Fig. 21 is a flow chart showing an example of a method for producing a nematode-encapsulated capsule in accordance with the present embodiment.

Description of Embodiments

[Nematode-encapsulated capsule]

A nematode-encapsulated capsule in accordance with the present embodiment includes an encapsulation composition containing a nematode and at least one layer made of membrane. In the present specification, a capsule is intended to mean at least one layer made of membrane which contains therein the encapsulation composition.

(Encapsulation composition)

The encapsulation composition contains a nematode. In the present specification, the term "nematode" encompasses nematodes in each developmental stage. The nematodes in each developmental stage include nematode eggs (including fertilized eggs), larvae (L1 larval stage to L4 larval stage), and adults. In the present specification, the adult nematodes include nematodes of each sex. The nematodes of each sex include male nematodes and hermaphroditism nematodes.

[Nematode]

The term "nematode" herein means both an animal belonging to *phylum Nematoda* and an animal belonging to *phylum Nematomorpha* according to the biological classifications. The nematode is not limited to any specific one, and may be any animal that is included in the above-described classifications, that is terrestrial or semiterrestrial, and that is able to move over a solid phase.

Examples of the animal belonging to *phylum Nematoda* encompass various kinds of nematodes such as nonparasitic nematodes (or free-living nematodes), plant-parasitic nematodes, entomogenous nematodes (including entomopathogenic nematodes, parasitoid nematodes, entomoparasitic nematodes, and phoretic nematodes), and nematodes parasitic on mammals (and the like).

Examples of the nonparasitic nematodes encompass *Caenorhabditis elegans* (which, hereinafter, may be referred to as "*C. elegans*"), *Aphelenchus avenae, Caenorhabditis angaria, Caenorhabditis brenneri, Caenorhabditis briggsae, Caenorhabditis japonica, Caenorhabditis remanei,* and *Pristionchus pacificus.*

Examples of the plant-parasitic nematodes encompass *Acreberoides nanus, Bastiania gracilis, Wilsonema othophorum, Meloidogyne incognita, Meloidogyne arenaria, Meloidogyne javanica, Meloidogyne hapla, Meloidogyne marytlandi, Meloidogyne mali, Heterodera glycines, Heterodera schachtii, Heterodera elachista, Globodera rostochiensis, Globodera pallida, Hirschmanniella diversa, Hirschmanniella immamuri, Pratylenchus penetrans, Pratylenchus coffeae, Pratylenchus vulnus, Pratylenchus neglectus, Pratylenchus loosi Loof, Pratylenchus curvitatus, Pratylenchus kumamotoensis, Pratylenchus pseudocoffeae, Ditylenchus dipsaci, Helicotylenchus dihystera, Helicotylenchus erythrinae, Hemicriconemoides kanayaensis, Paratrichodorus mirzai, Paratrichodorus minor (Colbran) Siddiqi* or *Trichodorus minor Colbran, Trichodorus porosus, Pratylenchus zeae Graham, Tetraopes annulatus, Tylenchorhynchus nudus, Aphelenchoides besseyi, Anguina tritici, Ditylenchus destructor, Aphelenchoides ritzemabosi, Longidorus spp., Xiphinema index, Aphelenchoides fragariae, Nothotylenchus acris, Pratylenchus brachyurus, Radopholus similis, Caenorhabditis inopinata,* and *Xiphinema brevicolle.*

Examples of the entomoparasitic nematodes encompass *Sphaerularia bombi, Sphaerularia vespae, Agamermis unka, Amphimermis zuimushi, Hexamermis microamphidis, Steinernema carpocapsae, Steinernema kushidai, Iotonchium ungulatum, Iotonchium californicum, Iotonchium cateniforme, Iotonchium laccariae, Iotonchium russulae, Caenorhabditis auriculariae, Bursaphelenchus tadamiensis, Contortylenchus sp., Contortylenchus genitalicola,* and *Romanomermis culicivorax.*

Examples of the phoretic nematodes of insects etc. encompass *Caenorhabditis japonica, Pristionchus pacificus, Bursaphelenchus xylophilus, Bursaphelenchus mucronatus, Bursaphelenchus doui, Bursaphelenchus firmae, Bursaphelenchus conicaudatus, Bursaphelenchus luxuriosae, Bursaphelenchus hunti, Bursaphelenchus okinawaensis, Bursaphelenchus yongensis, Bursaphelenchus kiyoharai, Bursaphelenchus cocophilus, Bursaphelenchus niphades, Bur-*

*saphelenchus sexdentati, Teratorhabditis synpapillata, Caenorhabditis briggsae,* and *Caenorhabditis remanei.* Among these, *Caenorhabditis japonica, Pristionchus pacificus, Caenorhabditis briggsae,* and *Caenorhabditis remanei* are dealt with as similar animals to the free-living nematodes (nonparasitic nematodes) in a laboratory.

Examples of the nematodes parasitic on mammals (and the like) encompass *Strongyloides stercoralis,* filariae, *Ascaridida, Anisakis,* whipworms, hookworms, *Gnathostoma,* and *Trichinella.*

Examples of the *Strongyloides stercoralis* are shown below for each taxonomy order of the primary host animals: *Strongyloides stercoralis* parasitic on animals of the order Anura of the class *Amphibia* (*Strongyloides pereira* (hereinafter, "*Strongyloides*", which is the generic name of the threadworm, may be abbreviated simply as "*S.*"), *S. carinii, S. amphibiophilus, S. bufonis, S. physali, S. spiralis, S. prokopici, S. mascomai,* etc.); *Strongyloides stercoralis* parasitic on animals of the order *Lacertilia* of the class *Reptilia* (*S. cruzi, S. darevskyi, S. ophiusensis,* etc.); *Strongyloides stercoralis* parasitic on animals of the order *Ophidia* of the class Reptilia (*S. ophidiae, S. mirzai, S. gulae, S. serpentis,* etc.); *Strongyloides stercoralis* parasitic on animals of the order *Ciconiiformes* of the class *Aves* (*S. cubaensis, S. ardeae, S. herodiae,* etc.); *Strongyloides stercoralis* parasitic on animals of the order *Galliformes* of the class *Aves* (*S. avium, S. oswaldoi, S. pavonis,* etc.); *Strongyloides stercoralis* parasitic on animals of the order *Anseriformes* of the class *Aves* (*S. minimum,* etc.); *Strongyloides stercoralis* parasitic on animals of the order *Charadriiformes* of the class *Aves* (*S. turkmenica,* etc.); *Strongyloides stercoralis* parasitic on animals of the order *Passeriformes* of the class *Aves* (*S. quiscali Barus,* etc.); *Strongyloides stercoralis* parasitic on animals of the order Marsupialia of the class Mammalia (*S. thylacis,* etc.); *Strongyloides stercoralis* parasitic on animals of the order *Insectivora* of the class Mammalia (*S. akbari, S. rostombekowi,* etc.); *Strongyloides stercoralis* parasitic on animals of the order *Primates* of the class *Mammalia* (*S. stercoralis, S. fuelleborni, S. fuelleborni kelly, S. cebus,* etc.); *Strongyloides stercoralis* parasitic on animals of the order Xenarthra of the class Mammalia (*S. dasypodis, S. shastensis,* etc.); *Strongyloides stercoralis* parasitic on animals of the *Pholidota* of the class *Mammalia* (*S. leiperi,* etc.); *Strongyloides stercoralis* parasitic on animals of the order *Rodentia* of the class *Mammalia* (*S. chapini, S. ratti, S. myopotami, S. venezuelensis, S. agoutii, S. robustus, S. sigmodontis,* etc.); *Strongyloides stercoralis* parasitic on animals of the order *Carnivora* of the class *Mammalia* (*S. nasua, S. felis, S. mustelorum, S. erschowi, S. planiceps, S. puttori, S. martis, S. vulpis, S. tumefasciens, S. lutrae, S. procyonis,* etc.); *Strongyloides stercoralis* parasitic on animals of the order *Proboscidea* of the class *Mammalia (S. elephantis,* etc.); *Strongyloides stercoralis* parasitic on animals of the order *Perissodactyla* of the class *Mammalia* (*S. westeri,* etc.); and *Strongyloides stercoralis* parasitic on animals of the order *Artiodactyla* of the class *Mammalia* (*S. papillosus, S. ransomi,* etc.). Among these, *S. stercoralis, S. fuelleborni,* and *S. fuelleborni kellyi* are so-called human *Strongyloides stercoralis,* which are parasitic to humans. In addition to them, *S. procyonis,* which is a raccoon *Strongyloides stercoralis,* and *S. ransomi,* which is a pig *Strongyloides stercoralis,* may sometimes be parasitic on humans.

Examples of the filariae encompass *Parafilaria multipapillosa, Stephanofilaria okinawaensis, Wuchereria bancrofti, Brugia malayi, Onchocerca cervicalis, Onchocerca gibsoni, Onchocerca gutturosa, Onchocerca volvulus, Acanthocheilonema reconditum, Setaria digitata, Setaria equina, Setaria labiatopapillosa, Setaria marshalli, Dirofilaria immitis,* and *Loa.*

Examples of the *Ascaridida* encompass *Ascaris lumbricoides, Ascaris suum, Baylisascaris transfuga, Lagochilascaris minor, Toxocara vitulorum* (or *Neoascaris vitulorum), Parascaris equorum, Baylisascaris procyonis, Toxocara canis, Toxocara cati, Toxascaris leonine,* and *Toxocara tanuki.*

Examples of *Anisakis* encompass so-called *Anisakis* type I, such as *Anisakis pegreffii, Anisakis simplex sensu stricto,* and *Anisakis simplex C, Anisakis* type II (also called *Anisakis physeteris), Anisakis typica, Psudoterranova decipiens,* and *Contracaecum osculatum.*

Examples of the whipworm encompass *Trichuris discolor, Trichuris muris, Trichuris ovis, Trichuris suis, Trichuris trichiura,* and *Trichuris vulpis.*

Examples of the hookworm encompass *Ancylostoma braziliense, Ancylostoma caninum, Ancylostoma ceylanicum, Ancylostoma duodenale, Ancylostoma kusimaense, Ancylostoma malayanum, Arthrostoma miyazakiense, Ancylostoma tubaeforme, Uncinaria stenocephala, Bunostomum phlebotomum, Bunostomum trigonocephalum, Globocepharus urosubulatus,* and *Necator americanus.*

Examples of *Gnathostoma* encompass *Gnathostoma nipponicum, Gnathostoma spinigerum, Gnathostoma hispidum, Gnathostoma doloresi, Gnathostoma procyonis,* and *Gnathostoma vietnamicum.*

Examples of the trichina encompass *Trichinella britovi, Trichinella spiralis, Trichinella nativa, Trichinella nelsoni,* and *Trichinella pseudospiralis.*

Examples of the nematodes parasitic on mammals (and the like) other than the above encompass *Abbreviata caucasica, Physaloptera praeputialis, Thelazia callipaeda, Thelazia rhodesi, Thelazia skrjabini, Habronema microstoma, Habronema muscae, Draschia megastoma, Crassicauda giliakiana, Gongylonema pulchrum, Ascarops strongylina,* and *Physocephalus sexalatus.* Further, the examples encompasses: strongyles such as *Strongylus asini, Strongylus edentates, Strongylus equinus,* and *Strongylus vulgaris;* and capillary nematodes (also referred to as *Capillaria*) such as *Eucoleus annulate, Eucoleus contorta, Pearsonema feliscati, Eucoleus perforans, Paracapillaria philippinensis* or *Capillaria philippinensis, Calodium hepatica* or *Capillaria aerophila, Aonchotheca bovis, Eucoleus aerophile,* and *Pearsonema*

*plica.* Furthermore, the examples encompasses: trichostrongyle nematodes such as *Trichostrongylus axei, Trichostrongylus colubriformis,* and *Trichostrongylus orientalis;* twisted stomach worms such as *Haemonchus contortus* and *Mecistocirrus digitatus; Dictyocaulus filarial; Dictyocaulus viviparous; Nematodirus filicollis; Angiostrongylus* such as *Angiostrongylus cantonensis, Angiostrongylus costaricensis,* etc.; and *Metastrongylus elongates* and *Filaroides hirthi.* Additionally, the examples encompasses pinworms such as *Enterobius vermicularis, Passalurus ambiguous, Syphacia muris, Syphacia obvelata,* and *Oxyuris equi.*

Further, examples of animals that belong to *phylum Nematomorpha* encompass horsehair worms (*Gordioidea*). Examples of *Gordioidea* encompass *Gordius robustus, Gordius ogatai, Pseudogordius tanganykae,* and *Chordodes japonensis.*

The nematodes to be encapsulated in the nematode-encapsulated capsule in accordance with the present embodiment are preferably *Caenorhabditis elegans* or a related species thereof. Examples of C. *elegans* or the related species thereof encompass *Caenorhabditis brenneri, Caenorhabditis briggsae, Caenorhabditis japonica, Caenorhabditis remanei,* and *Caenorhabditis inopinata.*

The above encapsulation composition may contain another component(s) in addition to the nematode. Examples of such another component(s) encompass: a water-soluble polymer that gelatinizes by reacting with a cation; a solution, such as water and a buffer solution, in which nematodes are viable; a culture solution of nematodes; food, such as *E. coli*, for nematodes; a culture solution for *E. coli*; food, such as yeast, for *E. coli*; a dye and a labeling substance, such as a fluorescent substance, for making it easy to visually observe the encapsulation composition; an antibiotic for preventing contamination with bacteria, fungi, and/or the like; and a hatching inhibitor (also referred to as "hatching suppressor"). Details of the water-soluble polymer will be described later.

By adjusting an amount of food, such as *E. coli,* in the encapsulation composition, it is possible to adjust a nutritional state of the nematode and to regulate a developmental stage or a growth rate of the nematode. Regulating the developmental stage or the growth rate of the nematode makes it easy to carry or store the nematode-encapsulated capsule for a medium-term to long-term period.

In a case where the developmental stage of the nematode to be encapsulated in the nematode-encapsulated capsule is an egg stage, development is arrested at the first larval stage larva (L1 larva) stage when the amount of food, such as *E. coli*, is small in the encapsulation composition. Such a nematode whose development has been arrested is called "dauer larva". The dauer larva can survive even in a state without nutrition for approximately one month. When an environmental condition such as the nutritional state is improved, the dauer larva returns to a normal developmental stage thereof and grow to be adult nematodes. Examples of the case where the amount of food, such as *E. coli*, in the encapsulation composition is small encompass a state in which, immediately after the nematode-encapsulated capsule is produced, the amount of food is not more than 10% or not more than 5% when the amount of eggs in the encapsulation composition is 100 mass%.

In a case where the developmental stage of the nematode to be encapsulated in the nematode-encapsulated capsule is the L1 larva stage, development is arrested at the L1 larva stage when the amount of food, such as *E. coli*, is small in the encapsulation composition. As a result, the nematode becomes a dauer larva. Examples of the case where the amount of food, such as *E. coli*, in the encapsulation composition is small encompass a state in which, immediately after the nematode-encapsulated capsule is produced, the amount of food is not more than 10% or not more than 5% when the amount of the LI larva in the encapsulation composition is 100 mass%.

Examples of the hatching inhibitor encompass: fluorodeoxyuridine (5-Fluoro-2'-deoxyuridine: FUdR, another name: Fluxuridine), which is a DNA synthesis inhibitor; and S,S-bis(1-methylpropyl)=O-ethyl-phosphorodithioate, another name: Cadusafos), which is an organic phosphorus insecticide that inhibits acetylcholine esterase activity. The hatching inhibitor makes it possible to suppress proliferation of the nematode after the nematode-encapsulated capsule is used, and consequently, to prevent diffusion of nematodes into the environment.

The number of nematodes in the encapsulation composition can be chosen as appropriate in accordance with an application, size, and the like of the nematode-encapsulated capsule. The encapsulation composition may contain one nematode (one egg in the case of an egg) or two or more nematodes (two or more eggs in the case of eggs).

The nematode may be a nematode having been exposed to radiation. Use of the nematode having been exposed to radiation makes it possible to adjust the percentage of eggs to be hatched. In addition, use of a larva having been exposed to radiation makes it possible to regulate development (e.g., arrest development, delay development, and/or the like). Such exposure to radiation will be described later.

Further, the nematode may be a nematode having been subjected to a hatching inhibitor treatment (typically, exposure). Use of an adult nematode having been exposed to a hatching inhibitor for a certain duration makes it is possible to adjust the number of eggs laid by the adult nematode or the percentage of the eggs to be hatched. The hatching inhibitor treatment will be described later.

Further, the nematode may be a nematode having been subjected to a sterilization treatment. Use of the nematodes having been subjected to the sterilization treatment makes it possible to suppress proliferation of the nematode after the nematode-encapsulated capsule is used, and consequently, to prevent diffusion of nematodes into the environment. The sterilization treatment will be described later.

(Membrane)

The membrane of the nematode-encapsulated capsule in accordance with the present embodiment is a membrane that forms an outer phase of the nematode-encapsulated capsule. The membrane is used to encapsulate therein the encapsulation composition containing the nematode (hereinafter, such a membrane may be referred to as "outer phase membrane"). The membrane contains, as a main component, a water-soluble polymer that gelatinizes by reacting with a cation. The phrase "containing, as a main component, a water-soluble polymer" herein means that, when a component excluding the liquid component in the membrane is 100 mass%, the water-soluble polymer contains not less than 50 mass%.

The membrane is preferably made of a material which is not edible for the nematode, a material that does not adversely affect the nematode, and/or a material having an oxygen permeability.

[Water-soluble polymer]

The water-soluble polymer that gelatinizes by reacting with a cation is preferably a water-soluble polymer that gelatinizes by reacting with a divalent cation (e.g., $Mg^{2+}$, $Ca^{2+}$). More preferably, the water-soluble polymer is a water-soluble polymer (calcium-coagulable polymer compound) that gelatinizes by reacting with a calcium ion.

Examples of the water-soluble polymer encompass proteins and polysaccharides. Specific examples of the water-soluble polymer encompass guar gum, casein, pectin, sodium carboxymethyl cellulose, sodium alginate, sodium polyacrylate, agar, kappa ($\kappa$) carrageenan, iota ($\iota$) carrageenan, lambda ($\lambda$) carrageenan, gluten, dextrin, xanthan gum, methylcellulose, starch, gelatin, locust bean gum, galactan, albumin, and keratin. The water-soluble polymer is preferably soluble in water at a temperature not higher than normal temperature. Among these, sodium alginate is more preferable. The water-soluble polymer may be any polymer that can be used in preparation at a low temperature, for example, a polymer that, although being soluble in hot water but insoluble in cold water, becomes soluble in cold water when formed into a sodium salt. Each kind of such water-soluble polymers can be used alone or two or more kinds of the water-soluble polymers can be used in combination.

The membrane may include another component(s) other than the water-soluble polymer. Examples of the another component, encompass: a dye; a labeling substance such as a fluorescent substance; a nematode; a component of a solution, such as water and a buffer solution, in which nematodes are viable; a component of a culture solution of nematodes; food, such as *E. coli,* for nematodes; a culture solution of *E. coli*; food, such as yeast, for *E. coli*; and an antibiotic for preventing contamination with bacteria, fungi, and/or the like. Inclusion of the dye or the labeling substance in the membrane makes it easy to visually observe an outer shape of the nematode-encapsulated capsule during visual observation or microscopic observation (e.g., fluorescence microscope observation).

The thickness and hardness of the membrane can be selected as appropriate in accordance with an application, the size, and the like of the nematode-encapsulated capsule. For example, reduction of the thickness of the membrane allows the nematode having been encapsulated to escape out of the nematode-encapsulated capsule by breaking the membrane.

In the nematode-encapsulated capsule in accordance with the present embodiment, the membrane may be made of a single layer or a plurality of layers.

(Shape of nematode-encapsulated capsule)

The nematode-encapsulated capsule in accordance with the present embodiment is not limited to any particular shape, provided that it is possible to encapsulate a desired amount of nematodes in the capsule. Examples of the shape of the capsule encompass a spherical shape, an elliptical shape, a tear drop shape, and a gourd shape. In addition, the capsule may have at least one flat surface.

The size of the nematode-encapsulated capsule in accordance with the present embodiment may be selected as appropriate in accordance with, for example, a type and a developmental stage (egg, larva, adult, or the like) of a nematode(s) to be encapsulated in the capsule, whether or not to subject, to a sterilization treatment, the nematode(s) prior to encapsulation into the capsule, sex, size and/or the number of the nematode(s). The capsule may have an outer diameter of several micrometers, tens of micrometers. or hundreds of micrometers. Alternatively, the capsule may have an outer diameter of several millimeters, tens of millimeters, or hundreds of millimeters. Moreover, the capsule may have an outer diameter of not more than 10 cm or not more than 1 cm. Further, the capsule may have an outer diameter of not less than 10 $\mu$m or not less than 20 pm. In the present specification, the outer diameter of the capsule refers to a maximum diameter of a cross section of the capsule.

Oxygen is essential for survival of the nematode encapsulated in the nematode-encapsulated capsule in accordance with the present embodiment. However, since the membrane of the capsule is permeable to oxygen, the nematode can survive in the capsule for several days. In a case where the encapsulation composition of the nematode-encapsulated capsule contains food, such as *E. coli*, for the nematode, the nematode can be grown inside the capsule.

In addition, even in a case where the nematode-encapsulated capsule in accordance with the present embodiment is put in a container such as a plastic dish and sealed, the nematode-encapsulated capsule can be stored in a good condition for several days. That is, the nematode-encapsulated capsule in accordance with the present embodiment can be transported, and the nematode encapsulated in the capsule can be used at a destination to which the nematode-encapsulated capsule has been transported. In addition, the nematode encapsulated in the capsule can be taken out by a simple operation such as making a hole in the capsule. Thus, use of the capsule allows even an untrained person to conduct an experiment or the like with use of the nematode.

In a case where the nematode-encapsulated capsule in accordance with the present embodiment is to be stored, a liquid such as water may be poured into a storage container. Injection of a liquid such as water into the storage container makes it possible to reduce an effect on the nematode that may be caused by vibration during transport.

In addition, increasing the number of nematode-encapsulated capsules in one storage container and storing the nematode-encapsulated capsules in a dense condition make it possible to reduce, e.g., rolling or damage of each of the nematode-encapsulated capsules that may be caused by vibration during transport. Alternatively, in a case where the nematode-encapsulated capsule is not spherical and has at least one flat surface, placing the nematode-encapsulated capsule in the storage container such that the flat surface comes at the bottom makes it possible to reduce rolling of the nematode-encapsulated capsule that may be caused by vibration and also to reduce the effect on the nematode in the capsule that may be caused by vibration during the transport.

(Method for producing nematode-encapsulated capsule)

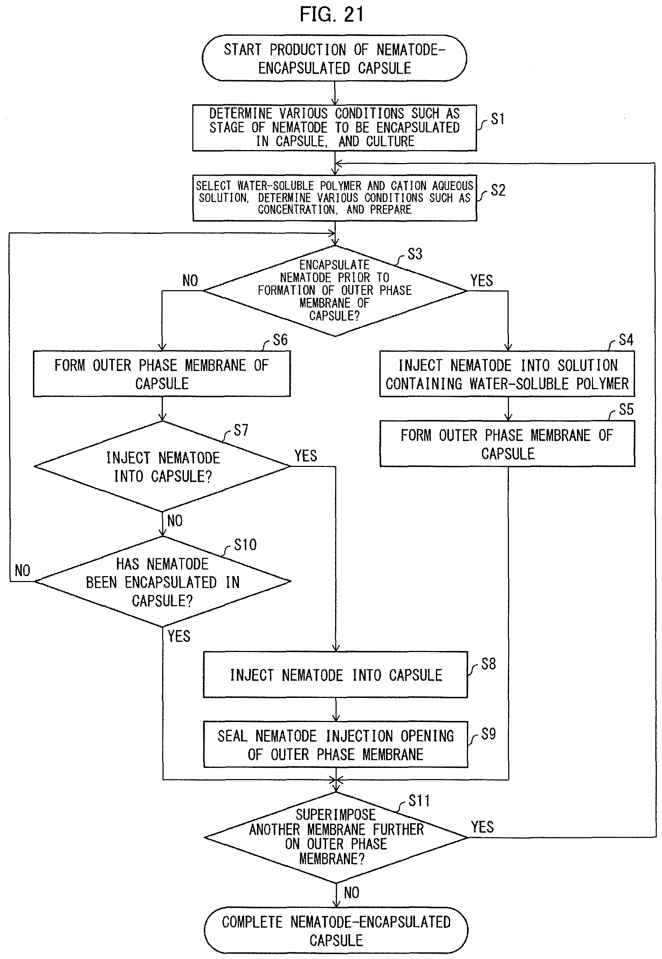

Examples of a method for producing a nematode-encapsulated capsule in accordance with the present embodiment encompasses: (1) a method in which a nematode is encapsulated at the same time as formation of a membrane that serves as an outer phase membrane of the capsule (which hereinafter may be referred to as "method (1)"); and (2) a method in which a nematode is injected after formation of the membrane that serves as the outer phase membrane of the capsule (which hereinafter may be referred to as "method (2)"). The following will describe these two methods with reference to a flow chart of Fig. 21.

[(S1) Determine various conditions such as developmental stage of nematode to be encapsulated in capsule, and culture nematode]

In this step, in accordance with an application and/or the like of the nematode-encapsulated capsule, determined are, for example, the type and the developmental stage (egg, larva, adult, or the like) of the nematode to be encapsulated, whether or not to subject, to a sterilization treatment, the nematode(s) prior to encapsulation, sex, and/or the number of nematodes to be encapsulated or an amount (volume) of a liquid containing a nematode(s) (which hereinafter may also be referred to as "nematode liquid"), and/or an amount of food. In order to obtain a nematode in a desired developmental stage, the nematode may be cultured prior to encapsulation.

(Sterilization treatment on nematode prior to encapsulation into nematode-encapsulated capsule)

Subjecting the nematode to be encapsulated in the nematode-encapsulated capsule to a sterilization treatment, it is possible to inhibit development of a next generation and to prevent proliferation of the nematode after use of the nematode in an experiment or the like or unnecessary diffusion of nematodes into the environment. The sterilization treatment can be carried out by a method using, for example, exposure to radiation or treatment with a chemical agent such as a hatching inhibitor, or a mutant that has a gonadal dysfunction. Such a method can be selected in accordance with a purpose.

(Sterilization treatment of nematode by exposure to radiation)

Exposing the nematode to radiation (X-rays, gamma rays, electron beams, heavy ion beams, proton beams, and/or the like) can inhibit the gonadal function of the nematode. The method for exposing the nematode to radiation may be selected in accordance with, for example, an irradiation facility, the type of nematode, the purpose of irradiation, and/or the like. Examples of exposure of the nematode to radiation encompass irradiation of a culture plate, irradiation of the culture solution put in a centrifuge tube, and irradiation in a state in which the nematode is encapsulated in a nematode retention microchip. Examples of exposure of a nematode to radiation encompasses: a method in which an entire body of the nematode is exposed to radiation by irradiation of an entire container containing the nematode; and a method in which only a gonad is exposed to radiation with use of a special irradiation device (e.g., a heavy-ion microbeam irradiation device) capable of irradiating a specific region of the nematode as a target.

A dose (hereinafter, referred to as "sterilization dose") necessary to cause all fertilized eggs that are born to a generation exposed to radiation to be unable to hatch varies depending on, for example, the type, the developmental stage or the sex of the nematode, or a type (quality) or a dose rate of the radiation. Sexes of nematodes are classified into female, female, and hermaphrodite. In a case where an individual exposed to radiation is a male, it is evaluated whether or not a next generation (fertilized egg) having been fertilized by mating after exposure to radiation with a female or a hermaphrodite having never been subjected to the sterilization treatment can hatch. In a case where the individual exposed to radiation is a female, it is evaluated whether or not a next generation (fertilized egg) having been fertilized by mating after exposure to radiation with a male having not been subjected to the sterilization treatment can hatch. On the other hand, in a case where the individual exposed to radiation is a hermaphrodite, it is evaluated whether or not a next generation (fertilized egg) that is self-fertilized before or after exposure to radiation can hatch or whether a next generation (fertilized egg) that is fertilized by mating after exposure to radiation with a male can hatch. For example, the following is reported: in a case where a nematode that is an adult hermaphrodite of C. *elegans* prior to laying eggs is exposed to radiation, $ED_{37}$, which is a dose (Effective Dose) that has the effect of reducing the hatching rate down to 37%, is approximately 184 Gray (Gy: unit of absorbed dose) for X-rays or gamma rays, and approximately 41 Gy for carbon ion beams, which are a kind of heavy ion beams. The threshold dose (sterilization dose: $ED_0$) at which the hatching rate becomes 0% is approximately 500 Gy for X-rays or gamma rays. Exposure of an adult nematode to a radiation dose lower than the sterilization dose allows for making adjustment so that only a certain percentage of eggs hatch. In addition, exposure of fertilized eggs or larvae to a radiation dose lower than the sterilization dose makes it possible to stop or delay development of nematodes. On the other hand, exposure of the nematode to a radiation dose much higher than the sterilization dose makes it possible to temporarily suppress movement of the nematode. This is advantageous in a case where the nematode-encapsulated capsule is used for the purpose of observation of the nematode.

(Sterilization treatment on nematode by treatment with chemical agent)

As a method for inhibiting (sterilizing) the gonadal function of the nematode, a chemical treatment can also be selected. In the chemical treatment, for example, the nematode is cultured on a medium to which fluorodeoxyuridine (hereinafter, referred to as "FUdR"), which is one type of hatching inhibitor, has been added, or the nematode is cultured on a culture solution containing FUdR.

A dose of a chemical agent required to make all eggs laid by a generation treated with the chemical agent unable to hatch varies depending on, for example, the type, the developmental stage or the sex of the nematode, a type of the chemical agent, or a treatment (exposure) time. For example, in a case where an FUdR solution is added to the culture plate and a nematode which is an adult hermaphrodite of *C. elegans* prior to laying eggs is exposed to the FUdR solution for a day, a sterilization dose at which the hatching rate of the next generation becomes 0% is not less than 0.5 mg/mL. Exposure of the adult nematode prior to laying eggs to the chemical agent of a dose lower than the sterilization dose allows for making adjustment so that only a certain percentage of eggs hatch.

(Hatching inhibitor addition treatment in encapsulating nematode)

In a case where a nematode to be encapsulated in the nematode-encapsulated capsule is a larva or an adult prior to laying eggs or an adult having not been treated with the hatching inhibitor, a chemical agent such as FUdR may be added to the encapsulation composition of the nematode-encapsulated capsule. In this case, it is possible to inhibit hatching of the eggs in the nematode-encapsulated capsule.

[(S2) Select water-soluble polymer and cation aqueous solution, determine conditions such as concentration, and prepare]

In this step, a water-soluble polymer such as a calcium-coagulable polymer compound and a cation such as a calcium salt solution are selected, and various conditions such as concentration are determined. Examples of the calcium-coagulable polymer compound encompass sodium alginate. Examples of a cation suitable for coagulation of the calcium-coagulable polymer compound encompass a calcium salt solution such as a calcium lactate aqueous solution or a calcium chloride aqueous solution. After the water-soluble polymer and the cation corresponding to the water-soluble polymer are selected, a solution containing the water-soluble polymer and a cation aqueous solution are prepared. The conditions such as the concentration may be determined on the basis of conditions that are used in a well-known method for producing so-called artificial particulates. For example, reference may be made to conditions that are disclosed in Japanese Patent Application Publication Tokukaihei No. 7 - 133209 and the like.

Adjustment of the concentration of the water-soluble polymer in the solution containing the water-soluble polymer allows for adjustment of the thickness and hardness of the membrane of the nematode-encapsulated capsule to be produced.

[(S3) Encapsulate nematode prior to formation of outer phase membrane of capsule?]

In this step, it is determined whether the nematode-encapsulated capsule is produced by the method (1) or the method (2).

[(S4) Inject nematode into solution containing water-soluble polymer]

This step is to be carried out in a case where the method (1) is selected in (S3). The encapsulation composition is prepared by injecting nematodes into the solution that was prepared in (S2) and that contains the water-soluble polymer. The solution that was prepared in (S2) and that contains the water-soluble polymer is sol. It is possible to prepare a nematode-containing sol by injecting the nematode liquid. In addition, one nematode may be collected at a time from the culture plate with use of a platinum wire picker, and be transferred to the aqueous sol. The number of nematodes to be put in the solution containing the water-soluble polymer or the amount (volume) of the nematode liquid can be selected as appropriate in accordance with the application, the size, and/or the like of the nematode-encapsulated capsule.

The flow chart in Fig. 21 shows an example in which the nematode liquid is injected into the solution containing the water-soluble polymer. However, the encapsulation composition may be prepared by adding the water-soluble polymer to the nematode liquid.

[(S5) Form outer phase membrane of capsule]

In this step, the encapsulation composition that was prepared in (S4) and that contains a nematode and a solution that was prepared in (S2) and that contains the cation are reacted with each other, so that a membrane that can serve as the outer phase membrane of the nematode-encapsulated capsule is formed. For example, by dropping or spraying the solution that was prepared in (S2) and that contains the cation in/on the encapsulation composition that was prepared in (S4) and that contains the nematode, the membrane that serves as the outer phase membrane of the nematode-encapsulated capsule is formed. Alternatively, by dropping or spraying the encapsulation composition that was prepared in (S4) in/on the solution that was prepared in (S2) and that contains the cation, the membrane that serves as the outer phase membrane of the nematode-encapsulated capsule is formed.

Adjustment of the amount (volume) of the encapsulation composition which is reacted with the solution that contains the cation makes it possible to adjust the size and shape of the nematode-encapsulated capsule.

In (a) a case where the solution that was prepared in (S2) and that contains the cation is dropped in the encapsulation composition that was prepared in (S4) and that contains the nematode or (b) a case where the encapsulation composition that was prepared in (S4) is dropped in the solution that was prepared in (S2) and that contains the cation, a dropped material is immersed in a receiving solution until after formation of the membrane, the membrane has a desired thickness and a desired hardness. Although it depends on a kind of a water-soluble biopolymer selected, the membrane becomes thicker and harder as an immersion time is longer (e.g., approximately 5 minutes), for example, in the case of a calcium-coagulable compound. In contrast, in a case where the immersion time is short (e.g., less than 30 seconds), the membrane is thin and soft. If the membrane is thin and soft, the nematode encapsulated in the nematode-encapsulated capsule can by itself break the membrane and escape out of the nematode-encapsulated capsule.

After the dropped material is immersed in the receiving solution, the dropped substance is taken out from the receiving solution. In order to prevent the membrane from further hardening, the dropped material may be washed with, for example, ultrapure water, and a receiving liquid component adhered to an outer side of the membrane may be removed.

In view of the simplicity of the operation and/or the like, it is preferable to drop the solution which contains the encapsulation composition that was prepared in (S4) in the solution that was prepared in (S2) and that contains the cation.

In a case where the solution that was prepared in (S2) and that contains the cation is dropped in the encapsulation composition that was prepared in (S4), reference can be made to Japanese Patent Application Publication Tokukaihei No. 5-92909 and/or the like. In a case where the encapsulation composition that was prepared in (S4) is dropped in the solution that was prepared in (S2) and that contains the cation, reference can be made to Japanese Patent Application Publication Tokukaihei No. 7-133209 and/or the like.

[(S6) Form outer phase membrane of capsule]

This step is to be carried out in a case where the method (2) is selected in (S3). The solution that was prepared in (S2) and that contains the water-soluble polymer and the solution that was prepared in (S2) and that contains the cation are reacted with each other, so that a membrane that can serve as the outer phase membrane of the capsule is formed. Formation in (S6) of the outer phase membrane of the capsule may be carried out in the same manner as in the formation in (S5) of the outer phase membrane of the capsule.

[(S7) Inject nematode into capsule?]

In this step, it is determined whether or not to inject a nematode into the capsule.

[(S8) Inject nematode into capsule]

This step is a step carried out in a case where injection of the nematode is selected in "(S7) Inject nematode into capsule?" Into the outer phase membrane of the capsule formed in (S6), a nematode is injected. The nematode can be injected by a method such as injection.

[(S9) Seal nematode injection opening in outer phase membrane]

In this step, after the nematode is injected in (S8), a nematode injection opening is sealed. The nematode injection opening which has been opened at the time of injection of the nematode can be sealed by, for example, a method in which a membrane for closing the injection opening is formed by dropping or spraying the cation aqueous solution after dropping or spraying a water-soluble polymer compound solution onto the injection opening.

[(S10) Has nematode been encapsulated in capsule?]

This step is a determination step carried out in a case where not to inject a nematode is selected in "(S7) Inject nematode into capsule?". In a case where the capsule is multilayered and a capsule in which a nematode is encapsulated has been prepared before formation of the outer phase membrane of the capsule in (S6), the step proceeds to (S11). In a case where the capsule is made of a single-layer outer phase membrane formed in (S6) shortly before or a case where the capsule is multilayered but no nematode is encapsulated in the capsule, the step returns to (S2) and production of the nematode-encapsulated capsule is continued.

[(S11) Superimpose another membrane further on outer phase membrane?]

In this step, in a case where a capsule obtained immediately before (S11) is single-layered, it is determined whether the membrane of the nematode-encapsulated capsule is completed as a single layer or another membrane is to be superimposed so as to form a multilayer. In a case where the capsule is multi-layered, it is determined whether or not another membrane is to be superimposed. In a case where it is determined that another membrane is to be superimposed, the capsule returns to (S2) and production of the nematode-encapsulated capsule whose membrane is multilayered is continued.

In the case of the nematode-encapsulated capsule including a multilayer membrane, the nematode may not be encapsulated in a membrane of the inner layer or may not be included in the encapsulation composition contained by the inner layer, but instead, may be encapsulated between the inner layer and an outer layer or encapsulated in the outer layer. Further, depending on an encapsulation position, the type, the developmental stage, the sex and/or the like of the nematode may be differed.

A method in accordance with the present embodiment for producing a nematode-encapsulated capsule produces a gel by reacting the water-soluble polymer that gelatinizes by reacting with a cation and the cation such as a calcium ion. Further, the membrane is formed on the surface of the gel. The encapsulation composition contained by the membrane is liquid or sol, so that the nematode can be encapsulated in the nematode-encapsulated capsule while fluidity of the liquid or sol is maintained.

In the method for producing the nematode-encapsulated capsule in accordance with the present embodiment, gelatinization is not induced by heating, and thus the nematode is not affected by a heat shock or the like. There is another advantage that the nematode is not chemically affected by the production process and the components of the nematode-encapsulated capsule. In addition, the membrane formed in the method for producing the nematode-encapsulated capsule in accordance with the present embodiment is oxygen permeable. Thus, the membrane can maintain oxygen supply, which is essential for the survival of the nematode encapsulated in the nematode-encapsulated capsule.

(Exposure of nematode-encapsulated capsule to radiation)

After the nematode-encapsulated capsule is produced, the capsule is exposed to radiation. This makes it possible to inhibit hatching of a nematode egg(s) that has been encapsulated or eggs that have been laid by an adult nematode inside the capsule. In this case, the nematode-encapsulated capsule may be put into a container and the entire container may be irradiated. As described above, the sterilization dose varies depending on, for example, the type, the developmental stage or the sex of the nematode, or the type or the dose rate of the radiation. Since the membrane of the nematode-encapsulated capsule may be broken down by exposure to radiation, it is desirable to avoid exposing the nematode-encapsulated capsule to a high dose which far exceeds the sterilization dose.

[Method for evaluating response of nematodes)

A method in accordance with the present embodiment for evaluating response of nematodes includes the steps of: supplying a nematode-encapsulated capsule to a nematode assay plate (which, hereinafter, may also be referred to simply as "plate"); discharging the nematodes from the nematode-encapsulated capsule; and evaluating response of the nematodes.

(Step of supplying nematode-encapsulated capsule to nematode assay plate)

This step is the step of supplying a nematode-encapsulated capsule to a nematode assay plate to a part of which a test substance has been provided (this step hereinafter may be referred to as "supply step").
The nematode assay plate is not limited to any particular plate, provided that the plate can be used in a response evaluation assay of nematodes. As an example, a nematode trap plate having a fall-in cavity (which, hereinafter, may also be referred to as "nematode trap plate") disclosed in Patent Literature 2 (International Publication No. WO 2020/218501) may be used. With use of the nematode trap plate, evaluation may be carried out in accordance with a method for evaluating response of nematodes, which is disclosed in Patent Literature 2.
As an alternative example, it is possible to use a nematode assay plate provided with a supply part for supplying nematodes. In this plate, the supply part is processed so as to suppress spread, on the plate, of a solution that contains the nematodes and that flows out from the nematode-encapsulated capsule (hereinafter, such a plate may be also referred to as "supply part-processed plate"). In a case where the supply part-processed plate is used, nematodes can be supplied by supplying the nematode-encapsulated capsule to the supply part. The following will describe the supply part-processed plate.

(Supply part-processed plate)

The supply part-processed plate includes, in a solid phase, a supply part to which the nematode-encapsulated capsule is to be supplied in the supply step. The supply part is processed for suppressing spread of a solution that contains the nematodes in the nematode-encapsulated capsule and that flows out from the nematode-encapsulated capsule in the step of discharging the nematodes from the nematode-encapsulated capsule, which will be described later. The processing for suppressing the spread makes it possible to keep the nematodes within a predetermined area and to sufficiently control the start position of movement of the nematodes. The processing is not limited to a specific aspect, provided that the processing is for suppressing the spread of the solution that contains the nematodes and that flows out from the nematode-encapsulated capsule. Examples of the processing encompass: making the supply part as a recessed part; and making a bottom surface (i.e., a surface with which the solution that contains the nematodes and that flows out from the nematode-encapsulated capsule comes in contact) hydrophobic. The following will describe a case where the solid phase is a solid medium, unless otherwise stated.
First, the following will explain making the supply part as a recessed part. By making the supply part as a recessed part, it is possible to retain, inside the recessed part, the solution that contains the nematodes and that flows out from the nematode-encapsulated capsule. This makes it possible to suppress the spread, on the plate, of the solution that contains the nematodes and that flows out from the nematode-encapsulated capsule. In a case where the nematodes stack on top of each other within the recessed part and form a massive group, the nematodes crawl out onto a surface of the solid medium in order from a nematode that is closest to an opening on a solid medium surface side of the recessed part. Thus, when the nematodes are released, it is possible to resolve the group of nematodes. For this reason, even in a case where a massive group of nematodes occurs, a state of the massive group can be prevented from being sustained since the recessed part is provided. In addition, since the nematode-encapsulated capsule can be kept within the recessed part, supply of the nematode-encapsulated capsule onto the plate becomes easy.
The recessed part is formed so as to extend from the surface of the solid medium toward the bottom of the solid medium. The depth of the recessed part is not limited to any particular depth, provided that the depth is within a range with which the effect of the recessed part can be obtained. For example, the recessed part may or may not reach the bottom surface of the solid medium.

The shape of the recessed part as viewed from an opening side of the recessed part not limited to any particular shape. In terms of easy observation of the nematodes in the recessed part under an upright microscope and easy crawling up of the nematodes onto the surface of the solid medium after drying of the solution in the nematode-encapsulated capsule, it is preferable that the opening of the recessed part has the same shape as the bottom surface of the recessed part or a shape that is wider than the bottom surface.

A method for forming the recessed part is not limited to any particular method. Examples of the method encompass: a method in which the recessed part is formed by hollowing out the solid medium; and a method in which the recessed part is formed by injecting a solid medium into a container having been provided in advance with a structure for forming the recessed part.

The volume of the recessed part is not limited to any particular volume. For example, in a case where the recessed part is formed by hollowing out a part of the solid medium, the volume of the recessed part is dependent on the thickness of the solid medium.

The container in which the solid medium is to be formed is not particularly limited in terms of size, shape, and material, and may be a commercially-available container, such as a plastic dish.

As the supply part-processed plate having the recessed part, for example, a solid medium having one recessed part (whose bottom surface area is 0.2 cm$^2$) having a cylinder shape of 5 mm in inner diameter may be formed in a circular plastic dish having a bottom surface inner diameter of approximately 8.4 cm and a bottom surface area of approximately 55 cm$^2$.

The recessed part may penetrate the solid medium, and the container may be exposed at the bottom surface of the recessed part. In a case where the bottom surface of the container is hydrophobic, a portion of the bottom surface is exposed in the recessed part and the solution that contains the nematodes and that has flown out from the nematode-encapsulated capsule is in contact with that hydrophobic portion. This can accelerate drying of the nematode liquid. The wording "being hydrophobic" herein refers to the property of repelling a liquid component of the solution that contains the nematodes and that flows out from the nematode-encapsulated capsule.

Since the bottom surface of the recessed part is hydrophobic in a case where the supply part is the recessed part, the nematode liquid supplied (dropped) to the bottom surface of the recessed part spreads throughout. Thereafter, the nematode liquid rapidly moves to a peripheral portion of the bottom surface of the recessed part, so that the center of the bottom surface of the recessed part is dried. Thus, the solution that contains the nematodes and that flows out from the nematode-encapsulated capsule can be efficiently moved to the peripheral portion of the bottom surface of the recessed part continuous with an inner wall of the recessed part, the inner wall becoming a movement line of the nematodes when the nematodes are crawling up to the surface of the solid medium. This makes it possible to suitably supply the nematodes to the surface of the solid medium.

In a case where the container is exposed at the bottom surface of the recessed part, making the entire container hydrophobic can be processing for making the bottom surface of the recessed part hydrophobic. In a case where the bottom surface of the recessed part is hydrophobic, the liquid component of the solution that contains the nematodes and that flows out onto the plate from the nematode-encapsulated capsule is repelled by the hydrophobic portion. Thus, the solution that contains the nematodes and that flows out from the nematode-encapsulated capsule can be rapidly moved from the center of the bottom surface of the recessed part to the peripheral portion of the bottom surface of the recessed part. In light of the above, the container is preferably made of a material that does not absorb liquid or that does not allow liquid to pass through the material. As such a material, it is possible to select one from, for example, the group consisting of plastic, glass, a silicone resin such as polydimethylsiloxane (PDMS), and a metal such as aluminum.

It is possible to obtain a similar effect by applying a hydrophobic material such as a water-repellent polymer to the bottom surface of the recessed part instead of exposing, at the bottom surface of the recessed part, a part that is made of a hydrophobic material and that constitutes the container. In a case where the hydrophobic material such as a water-repellent polymer is applied to the recessed part, the hydrophobic material may be applied not only to the bottom surface but also to the inner wall (lateral surface) of the recessed part.

Note that, in a case where the supply part other than the recessed part is provided, the processing for making the surface of the supply part hydrophobic is not particularly limited. Examples of the processing encompass processing in which a hydrophobic sheet having a desired material and shape may be placed on a nematode assay plate, and a top of the sheet is used as the supply part.

In a case where the supply part is a recessed part, the inner wall of the recessed part for connecting the bottom surface and the opening of the recessed part may be water-absorbing. In a case where the inner wall of the recessed part is water-absorbing, the liquid component of the solution that contains the nematodes and that flows out from the nematode-encapsulated capsule is absorbed in the solid medium. This makes it possible to accelerate drying of the nematode liquid in the recessed part.

In the present embodiment, the wording "being water-absorbing" refers to the property of the solid medium absorbing a liquid component of the solution (not limited to water) that flows out from the nematode-encapsulated capsule. For example, the wording indicates that the solid medium absorbs not less than 40%, preferably not less than 50%, and more preferably not less than 70% of the liquid component of the solution that contains the nematodes and that flows out from the nematode-encapsulated capsule, within approximately 30 minutes from supply of the solution.

The recessed part may be arranged such that the bottom surface is hydrophobic and the inner wall is water-absorbing. This configuration allows the solution that contains the nematodes and that flows out from the nematode-encapsulated capsule to more suitably move to the peripheral portion of the bottom surface of the recessed part. Thus, since the liquid component of the solution that contains the nematodes and that flows out from the nematode-encapsulated capsule is more suitably absorbed by the inner wall of the recessed part, it is possible to reduce a drying time of the solution.

A method for making the inner wall of the recessed part water-absorbing is not limited to any particular method. For example, by forming the recessed part by hollowing out the solid medium, the inner wall of the recessed part may be made water-absorbing. When the recessed part is formed by hollowing out the solid medium, fine holes are formed inside the recessed part as the solid medium is solidified, and are exposed on the inner wall of the recessed part. Then, it is possible to make the inner wall of the recessed part water-absorbing by action of those holes exposed.

Note that compared to a recessed part formed by a method in which a solid medium is poured and solidified in a container having been provided in advance with a structure for forming the recessed part, for example, a container to/in which a protruding material is attached or inserted, the inner wall of the recessed part formed by the method in which the solid medium is hollowed out has a rougher inner surface and a larger area. It is considered that, by forming the recessed part by hollowing out as described above, the recessed part has a larger area and a larger number of fine holes where the liquid component of the solution that contains the nematodes and that flows out from the nematode-encapsulated capsule can be absorbed. For this reason, it is preferable that the recessed part is formed by hollowing out the solid medium.

The area of the inner wall of the recessed part is not limited to any particular area. In order to enhance a water-absorbing property for the nematode liquid at the inner wall of the recessed part, the area of the inner wall of the recessed part is preferably not less than 0.1 cm$^2$ but not more than 4 cm$^2$, and more preferably not less than 0.2 cm$^2$ but not more than 2 cm$^2$.

The larger the area of the aforesaid water-absorbing inner wall of the recessed part is, the better the water-absorbing property becomes. However, in a case where the area of the inner wall of the recessed part is increased due to an increase in amount of the solid medium, drying of the liquid component of the solution that contains the nematodes and that flows out from the nematode-encapsulated capsule is reduced. This is, for example, because in this case, humidity increases in the supply part-processed plate, and thus in the recessed part. Further, the time required to dry the liquid component of the solution that contains the nematodes and that flows out from the nematode-encapsulated capsule is affected by, for example, the following various conditions: the size and shape of the supply part-processed plate; the size, shape, and number of recessed parts; the amount (volume) of the solution that contains the nematodes and that flows out from the nematode-encapsulated capsule; the amount (volume) of the solid medium; and nematode population density. Thus, the area of the inner wall of the recessed part may be selected as appropriate with reference to the above-described ranges and other conditions.

The depth of the recessed part is preferably not less than 0.1 cm but not more than 1 cm, and more preferably not less than 0.14 cm but not more than 0.6 cm, in order to suitably release the nematodes. In a case where the depth of the recessed part is not lower than, particularly, the lower limit, the area of the inner wall of the recessed part can be set within a sufficient range. This makes it possible to sufficiently maintain the water-absorbing property of the inner wall of the recessed part. Further, in a case where the depth of the recessed part is not higher than, particularly, the upper limit, it is possible to reduce a distance of movement of the nematodes before crawling out from the recessed part and to control the humidity inside the recessed part within a suitable range. This makes it possible to reduce the time taken before the nematodes are released.

The depth of the recessed part may be set small by having a relatively large amount (volume) of the solid medium poured in the supply part-processed plate, that is, having a relatively small thicknesses of the solid medium. This reduces the distance from the bottom surface of the recessed part to the surface of the solid medium. This makes it possible to limit the number of nematodes stacked along the inner wall from the bottom surface of the recessed part toward the opening on the solid medium surface side. Accordingly, the size of the group of nematodes can be reduced. In addition, since a distance by which the nematodes crawl up on the inner wall of the recessed part is reduced, the release of the nematodes becomes smoother. For example, in a case where the depth of the recessed part is not less than 0.17 cm but not more than 0.25 cm, it is possible to start release of the nematodes within 3 minutes after discharging the nematodes from the nematode-encapsulated capsule.

The amount (volume) of the solid medium may be varied as appropriate depending on a method of preparing the solid medium, the size of the container, and conditions of a drying step, which will be described later. The following will describe the amount of the solid medium for a case where the supply part is arranged as a recessed part, using, as an example, a solid medium obtained by pouring a medium into a container and solidifying the medium, and further leaving the medium alone for a whole day and night in a tightly sealing storage container at room temperature. Note that since the moisture content of the solid medium changes due to the length of a storage time after solidification, the conditions of the supply step and the drying step, which will be described later, may be adjusted in accordance with a state of the solid medium.

The amount (volume) of the solid medium is set as a rule to a thickness at which the solution that contains the nematodes and that flows out from the nematode-encapsulated capsule supplied to the recessed part does not flow over onto the surface of the solid medium. The thickness is not limited to a particular thickness, provided that the thickness is within a range within which an assay can be suitably conducted. For example, in view of suitably drying the liquid component of the solution that contains the nematodes and that flows out from the nematode-encapsulated capsule, the amount of the solid medium is set to preferably not more than 40% of the volume of the container. The lower limit is not particularly set. However, if the amount of the solid medium is not less than 10%, the solution that contains the nematodes and that flows out from the nematode-encapsulated capsule does not flow over to the solid medium and the assay can be conducted in such a range. In order to set the amount of the solid medium within the range, for example, in a case where a circular dish having a bottom surface inner diameter of approximately 8.4 cm and a volume of approximately 72 $cm^3$ is used, the amount of the solid medium may be approximately 10 mL to 30 mL, that is, a thickness of approximately 0.2 cm to 0.6 cm. Alternatively, in a case where a circular dish having a bottom surface inner diameter of approximately 5.2 cm and a volume of approximately 21 $cm^3$ is used, the amount of the solid medium may be approximately 3 mL to 10 mL, i.e., a thickness of approximately 0.14 cm to 0.5 cm.

Note that in a case where the recessed part is formed by hollowing out the solid medium, the depth of the recessed part is equal to the thickness of the solid medium and dependent on the amount (volume) of the solid medium. Thus, the depth of the recessed part may be adjusted so as to be within the range described above.

The amount (volume) of the solid medium may be set to be not more than 20% of the volume of the container. In a case where the other conditions such as the amount of the solution that contains the nematodes and that flows out from the nematode-encapsulated capsule supplied are the same, the liquid component of the solution that contains the nematodes and that flows out from the nematode-encapsulated capsule supplied is dried at a faster rate when the solid medium within the above range is used than when the solid medium of an amount larger than the range is used. On this account, in a case where it is desired that the time required for the drying step described later is relatively short, the solid medium within the above range can be suitably used. Note that the wording "the time required for the drying step is relatively short" in the present embodiment means that typically, the time required for the drying step is within 25 minutes. In order to set the amount of the solid medium within the range, for example, in a case where a circular dish having a bottom surface inner diameter of approximately 8.4 cm and a volume of approximately 82 $cm^3$ is used, the amount of the solid medium should be approximately 10 mL, that is, the thickness should be approximately 0.2 cm to 0.4 cm. Alternatively, in a case where a circular dish having a bottom surface inner diameter of approximately 5.2 cm and a volume of approximately 25 $cm^3$ is used, the amount of the solid medium should be approximately 3 mL, i.e., the thickness should be approximately 0.15 cm.

In accordance with the assay, the amount (volume) of the solid medium may be set to not more than 40% of the volume of the container. There is no upper limit for the amount of the solid medium. However, in order to facilitate the assay, the volume of the solid medium is preferably not more than 50% of the volume of the container. In a case where the other conditions such as the amount (volume) of the solution that contains the nematodes and that flows out from the nematode-encapsulated capsule supplied are the same, the liquid component of the solution that contains the nematodes and that flows out from the nematode-encapsulated capsule is dried at a slower rate when the solid medium within the above range is used than when the solid medium of an amount smaller than the range is used. On this account, in a case where it is desired that the time required for the drying step is relatively long, the solid medium within the above range can be suitably used. Note that the wording "the time required for the drying step is relatively long" in the present embodiment means that typically, the time required for the drying step is not less than 60 minutes but not more than 180 minutes. Note that, in a case where a time exceeding 180 minutes is required, the nematodes to be used in the assay may be in a starving state, so that the nematodes may become unsuitable for a normal assay. However, in an assay in which starving nematodes are to be used, it is possible to choose drying that requires an extremely long time that exceeds 180 minutes.

The number, shape, size, and formation position of the supply parts are not particularly limited. Such conditions may be changed as appropriate in accordance with the type and number of nematodes to be used, the type and number of test substances to be used, the purpose of an assay, and the like. The number of supply parts can be, for example, 1, 2, 3, 4, 5, 6 or more. The shape of each of the supply parts may be, for example, circular, rectangular, of polygonal in a cross section obtained when the supply part is horizontally cut.

The size of the supply parts is preferably sufficiently small relative to the size of the supply part-processed plate. For example, in a case where the supply parts are arranged as recessed parts, the total area of openings on a plate surface side of the recessed parts may be not more than approximately 2%, and preferably not more than 0.4% of the surface of the solid medium. In a case where the area of the openings of the recessed parts is within the above range, it is possible to suitably suppress, without hampering performance of the assay, the spread on the plate of the solution that contains the nematodes and that flows out from the nematode-encapsulated capsule. Further, the position of the supply part is typically in the vicinity of the center of the supply part-processed plate. However, the position of the supply part is not limited to any particular position, and may be set according to the purpose and content of the assay.

The size of the container to be used for the supply part-processed plate is not limited to any particular size, provided that the size is suitable for the assay. For example, in the case of a circular container, the container has preferably an inner diameter of the bottom surface of not less than 3.0 cm but not more than 15.0 cm and a height (inner height) of not less than 0.5 cm but not more than 3 cm, and more preferably, an inner diameter of not less than 5.0 cm but not more than 10.0 cm and a height (inner height) of not less than 1 cm but not more than 1.5 cm. In the case of a rectangular container, the container has inside dimensions of not less than 3 cm but not more than 30 cm in depth (typically, short side), not less than 3 cm but not more than 30 cm in width (typically, long sides), and not less than 0.5 cm but not more than 3 cm in height, and more preferably, not less than 5 cm but not more than 15 cm in width, not less than 5 cm but not more than 25 cm in width, and not less than 1 cm but not more than 1.5 cm in height. In a case where the inner dimensions of the bottom surface of the container are in the above ranges, it is possible to suitably form the following various gradients inside the supply part-processed plate: an odor gradient of a test substance in a chemotaxis assay concerning olfactory sense; a concentration gradient of salinity or the like in a chemotaxis assay concerning taste sense; a temperature gradient in a thermotaxis assay; and/or the like. In addition, the volume of the container is preferably not less than 3.6 $cm^3$ but not more than 2700 $cm^3$, and preferably not less than 19.5 $cm^3$ but not more than 565 $cm^3$.

(Step of discharging nematodes from nematode-encapsulated capsule)

This step is to carry out the process of discharging the nematodes from the nematode-encapsulated capsule supplied by the above-described supply step (this step hereinafter may be referred to as "discharge step"). For example, the nematodes may be discharged by forming a hole in the nematode-encapsulated capsule. After the nematodes are discharged, a solution containing the nematodes may be pipetted or the like so that the density of the nematodes becomes constant.

In a case where a supply part-processed plate is used, the drying step may be carried out after the discharge step. In the drying step, the liquid component of the solution that contains the nematodes and that has flown out from the nematode-encapsulated capsule is dried, so that the nematodes easily move on the plate. The liquid component of the solution that contains the nematodes and that has flown out from the nematode-encapsulated capsule may be dried by, for example, evaporation of the solution. By carrying out the drying step, the liquid component of the solution that contains the nematodes and that has flown out from the nematode-encapsulated capsule is dried. Consequently, the nematodes easily move, and the nematodes are released onto the supply part-processed plate. In a case where the supply part is processed for suppressing spread on the plate of the solution that contains the nematodes and that flows out from the nematode-encapsulated capsule, the solution that contains the nematodes can be prevented from spreading until the liquid component of the solution dries. This can keep the nematodes within a predetermined area. This makes it possible to regulate the timing of releasing the nematodes, in particular, in the case of using a nematode-encapsulated capsule having an outer diameter of several centimeters and having a large amount (volume) of the encapsulation composition. In the present specification, the term "release" means that the liquid component of the solution that contains the nematodes and that flows out from the nematode-encapsulated capsule is dried and the nematodes crawl out onto the supply part-processed plate. In the present embodiment, the term "drying" means that the liquid component of the solution that contains the nematodes and that has flown out from the nematode-encapsulated capsule is removed to an extent that the nematodes can freely move on the supply part-processed plate. Typically, the term "drying" means a state in which not less than 80% of the liquid component is removed.

(Method for evaluating response of nematodes)

This step is to evaluate response (e.g., taxis) of the nematodes discharged in the discharge step with respect to a test substance (this step hereinafter may be referred to as "response evaluation step").

Nematodes exhibits chemotaxis that the nematodes are attracted to a specific concentration of sodium chloride (NaCl), chemotaxis that the nematodes escape from a specific concentration of diacetyl, taxis that the nematodes are attracted to urine of a cancer patient; and taxis (thermotaxis) to a specific temperature.

For example, in a case where the test substance is urine collected from a subject, the possibility that the subject has cancer can be determined by the response evaluation step. That is, the method in accordance with the present embodiment for evaluating response of nematodes can be used in a cancer test method. The cancer test may include, for example, the steps of: supplying a target test substance to a part of a nematode assay plate (forming a concentration gradient of the target test substance); supplying the nematode-encapsulated capsule to a predetermined position; discharging the nematodes from the nematode-encapsulated capsule; and after lapse of a predetermined time period, (a) counting the number of nematodes that are attracted to the test substance or nematodes that have escaped from the test substance or (b) measuring an element co-related with the number of nematodes. The nematode assay plate is not limited to any particular plate, provided that the plate can be used in an assay for evaluating response of the nematodes to cancer. The above-described nematode trap plate may be one examples of the nematode assay plate. With use of the nematode trap plate, evaluation may be carried out in accordance with a cancer test method disclosed in Patent Literature 2. The number of nematodes attracted to the test substance may be, for example, the number of nematodes present in a predetermined region from the part of the nematode assay plate. In addition, the number of nematodes that have escaped from the part of the nematode assay plate may be the number of nematodes present outside the predetermined region from that part.

Examples of the test substance to be used in the cancer test method encompass a biological sample collected from a subject. Examples of the biological sample encompasses: body fluids such as urine, sweat, saliva, pleural effusion, ascites fluid, stool, and blood (including individual components separated from blood, e.g., plasma, serum, and the like); cancer tissues such as cells (e.g., biopsy cells), biopsy tissues, and tissue sections; and storage solution, culture solution, and the like of biological tissues. The test substance is preferably a body fluid that can be easily collected from a mammal to be tested, and more preferably urine or blood.

Further, the method in accordance with the present embodiment for evaluating response of the nematodes may be used to conduct an olfaction assay of the nematodes with respect to a test substance that emits an odorant or a gustation assay of the nematodes with respect to a test substance that has flavor. These chemotaxis assays may include the steps of: supplying a target test substance to a part of a nematode assay plate (forming a concentration gradient of the target test substance); supplying the nematode-encapsulated capsule to a predetermined position; discharging the nematodes from the nematode-encapsulated capsule; and after lapse of a predetermined time period, (a) counting the number of nematodes that are attracted to the test substance or nematodes that have escaped from the test substance or (b) measuring an element co-related with the number of nematodes. For example, through the olfaction assay, an assay for electing an antagonistic or repellent animal against the plant-parasitic nematodes can be carried out. The nematode assay plate is not limited to any particular plate, provided that the plate can be used in a chemotaxis assay of nematodes. With use of the nematode trap plate, evaluation may be carried out in accordance with a method for evaluating response of nematodes, which is disclosed in Patent Literature 2. The number of nematodes attracted to the test substance may be, for example, the number of nematodes present in a predetermined region from the part of the nematode assay plate. In addition, the number of nematodes that have escaped from the part of the nematode assay plate may be the number of nematodes present outside the predetermined region from that part.

Furthermore, an assay for examining response (thermotaxis) of nematodes to a temperature may be carried out with use of the method for evaluating response of the nematodes in accordance with the present embodiment. The thermotaxis assays include the steps of: adjusting a part of the nematode assay plate to a target temperature; supplying the nematode-encapsulated capsule to a predetermined position; discharging the nematodes from the nematode-encapsulated capsule; and after lapse of a predetermined time period, (a) counting the number of nematodes that are attracted to the part of the nematode assay plate or nematodes that have escaped from the part or (b) measuring an element co-related with the number of nematodes. The nematode assay plate is not limited to any particular one, provided that it can be used in a thermotaxis assay of nematodes. With use of the nematode trap plate, evaluation may be carried out in accordance with a method for evaluating response of nematodes, which is disclosed in Patent Literature 2. The number of nematodes attracted to the part of the nematode assay plate may be, for example, the number of nematodes present in a predetermined region from the part of the nematode assay plate. In addition, the number of nematodes that have escaped from the part of the nematode assay plate may be the number of nematodes present outside the predetermined region from that part.

(Nematode response evaluation kit)

A nematode response evaluation kit in accordance with the present embodiment includes the nematode-encapsulated capsule. In addition to the capsule, the nematode assay plate may be included. The nematode assay plate is not limited to any particular plate, provided that the plate can be used in a response evaluation assay of nematodes. Examples of the nematode assay plate encompass a supply part-processed plate on which the above-described supply part is formed and a nematode trap plate. Further, the evaluation kit may include written instructions in which procedures etc. for evaluating response of nematodes are described. The written instructions may be printed on paper or written in another medium or alternatively may be attached to an electronic medium such as a magnetic tape, a disc that is readable by a computer or the like, or a CD-ROM. Instead of the written instructions, an audio file or video file may be included that describes the procedures etc. for evaluating response of nematodes. The audio file or video file may be attached to an electronic medium such as a magnetic tape, a disc that is readable by a computer or the like, or a CD-ROM, or may be in a format in which such a file is stored in an accessible cloud server or the like and referred to via an internet line.

As described above, nematodes have the property (a kind of taxis) of being attracted to urine of a cancer patient, and therefore, the nematode response evaluation kit in accordance with the present embodiment may be used as a cancer test kit.

(Production kit for producing nematode-encapsulated capsule)

A production kit for producing the nematode-encapsulated capsule in accordance with the present embodiment includes a nematode, a water-soluble polymer, and a salt of a cation. The nematode may be a nematode having been subjected to a treatment of exposure to radiation, a treatment of exposure to a hatching inhibitor, or another sterilization treatment. Examples of the aqueous polymer encompass alginate salts such as sodium alginate. Examples of the cation encompass calcium salts such as calcium chloride and calcium lactate. The production kit may include a hatching inhibitor (e.g., powder or liquid) or a liquid containing a chemical agent.

Further, the production kit may include written instructions in which procedures etc. for producing the nematode-encapsulated capsule are described. The written instructions may be printed on paper or written in another medium or alternatively may be attached to an electronic medium such as a magnetic tape, a disc that is readable by a computer or the like, or a CD-ROM. Instead of the written instructions, an audio file or video file may be included that describes the procedures etc. for producing the nematode-encapsulated capsule. The audio file or video file may be attached to an electronic medium such as a magnetic tape, a disc that is readable by a computer or the like, or a CD-ROM, or may be in a format in which such a file is stored in an accessible cloud server or the like and referred to via an internet line.

With the above-described nematode-encapsulated capsule in accordance with the present embodiment and techniques using the nematode-encapsulated capsule, an experimenter who has no technique or facility for culturing nematodes required for experiments or the like also can easily conduct the experiments. Specifically, a nematode can be supplied to, for example, a nematode assay plate at the time of conducting an experiment only by causing the nematode to escape from inside the capsule by any of the following operations: an operation to pick and break the membrane of the nematode-encapsulated capsule with use of a tip of a toothpick, chip, or the like; an operation to add a chelator, such as a solution of sodium citrate, and dissolve the membrane; and an operation to bring the nematode-encapsulated capsule in contact with a mixture of a microorganism, a chemical substance, and the like (typically, scattering on soil). For example, the use of the nematode-encapsulated capsule in accordance with the present embodiment is effective in a space experiment which requires reduction of operations of an experimenter and reduction in a test sample storage space. In addition, the use of the nematode-encapsulated capsule in accordance with the present embodiment is effective even in a laboratory scene in which an experimenter himself or herself is assumed to have no technique or facility for culturing nematodes, such as an experiment (experience) conducted in school education, at home, or the like. That is, in a space experiment or a scene in which an experimenter who is not an untrained person conducts an experiment, use of nematodes in a good condition suitable for the experiment or the like becomes possible in a simple operation by acquiring the nematode-encapsulated capsule in accordance with the present embodiment. Furthermore, in a scene in which an agricultural worker or pest nematode exterminator who is an untrained person for nematode culture or the like discharges desired nematodes in an environment for the purpose of pest nematode control or the like, the desired nematodes can be used by a simple operation by obtaining the nematode-encapsulated capsule in accordance with the present embodiment.

In addition, a nematode-encapsulated capsule in which a nematode is encapsulated in a good condition contributes to (i) cases where it is desired to observe the nematode in the capsule without breaking or dissolving the membrane or (ii) efficiently conducting an experiment in which keeping the nematode at a predetermined position is effective (e.g., an ion microbeam irradiation experiment in which radiation is emitted to a target range of several tens of $\mu$m in a certain tissue).

In a case where the nematode-encapsulated capsule is to be used in an experiment (experience) conducted in school education, at home, or the like, it is preferable to inhibit proliferation of a nematode(s) in the nematode-encapsulated capsule after use so as to prevent diffusion of nematodes into the environment. In this regard, it is preferable to use, in the experiment (experience) conducted in school education, at home, etc., the nematode-encapsulated capsule in which a nematode having been subjected to a sterilization treatment is encapsulated.

In addition, it is assumed that pest nematodes that cause great damage to agricultural crops or a tree(s) are controlled by (i) scattering, on soil, nematode-encapsulated capsules in each of which, for example, a pest nematode having been subjected to a sterilization treatment, a predatory nematode of the pest nematode, or the like is contained or (ii) injecting the nematode-encapsulated capsules in the tree (sterile insect release). In this case, for example, the nematode-encapsulated capsules are scattered on the soil or tree, and then mixed or buried in the soil or tree. Then, the capsules are left alone, so that the membrane of the capsules can be slowly degraded over several hours or days by microorganisms, a chemical substance, and/or the like contained in the soil or the tree. This makes it possible to discharge the nematodes. In order to prevent diffusion of more nematodes than necessary into the environment, it is preferable to use nematode-encapsulated capsules in which the nematodes having been subjected to a sterilization treatment are encapsulated.

Furthermore, a technique for supplying nematodes for assays in a good condition is essential in order to make a "simple assay kit" available as a product for conducting, for example, at a medical institute, at home, or in mass screening at the workplace or in community, a cancer test that uses nematodes and that is presently conducted only at a specialized laboratory. Containment and culture of a nematode(s) in a nematode-encapsulated capsule according to an aspect of the present invention make it possible to supply the nematodes in a good condition at low cost. In a cancer test using nematodes, a nematode response evaluation assay, or the like, a technique disclosed in a published patent application publication (e.g., Patent Literature 2) related to the nematode assay plate, the nematode assay method, and/or the like realized by the present inventor can be used in combination.

Use of a cancer test using the nematode-encapsulated capsule in accordance with the present embodiment makes it possible to conduct simply and regularly a cancer test. This can enhance measures for early detection of a risk factor that may damage health, mitigate the risk factor, and avoidance of the risk factor. This can lead to extension of healthy life expectancy and can contribute to achieving a Sustainable Developmental Goals (SDGs).

Aspects of the present invention can also be expressed as follows:
A nematode-encapsulated capsule according to Aspect 1 of the present invention includes: an encapsulation composition containing a nematode; and at least one layer made of membrane with which the encapsulation composition is encapsulated, the membrane containing, as a main component, a water-soluble polymer that gelatinizes by reacting with a cation.

A nematode-encapsulated capsule according to Aspect 2 of the present invention may be configured, in the above Aspect 1, to have an outer diameter of not more than 10 cm.

A nematode-encapsulated capsule according to Aspect 3 of the present invention may be configured, in the above Aspect 1 or 2, such that the nematode is *Caenorhabditis elegans* or a related species thereof.

A nematode-encapsulated capsule according to Aspect 4 of the present invention may be configured, in any one of the above Aspects 1 to 3, such that the water-soluble polymer is at least one kind of water-soluble polymer selected from the group consisting of proteins and polysaccharides.

A nematode-encapsulated capsule according to Aspect 5 of the present invention may be configured, in any one of the above Aspects 1 to 4, such that the nematode is a nematode having been exposed to radiation.

A nematode-encapsulated capsule according to Aspect 6 of the present invention may be configured, in any one of the above Aspects 1 to 5, such that the nematode is a nematode having been treated with a hatching inhibitor.

A nematode-encapsulated capsule according to Aspect 7 of the present invention may be configured, in any one of the above Aspects 1 to 6, such that the nematode is a nematode having been subjected to a sterilization treatment.

A nematode-encapsulated capsule according to Aspect 8 of the present invention may be configured, in any one of the above Aspects 1 to 7, the encapsulation composition further contains a hatching inhibitor.

A method according to Aspect 9 of the present invention for producing a nematode-encapsulated capsule is a method for producing the nematode-encapsulated capsule according to any one of Aspects 1 to 8, the method including the steps of: preparing the encapsulation composition by mixing the nematode and the water-soluble polymer with each other; and producing the nematode-encapsulated capsule by reacting the encapsulation composition and a solution containing the cation.

A method according to Aspect 10 for producing a nematode-encapsulated capsule is a method for producing the nematode-encapsulated capsule according to any one of Aspects 1 to 8, the method including the steps of: preparing a base material of the membrane of the nematode-encapsulated capsule by reacting a solution containing the water-soluble polymer and a solution containing the cation; and producing the nematode-encapsulated capsule by injecting the nematode into the base material.

A method according to Aspect 11 of the present invention may be configured in the above Aspect 9 to further include the step of exposing the nematode-encapsulated capsule to radiation.

A method according to Aspect 12 of the present invention may be configured in the above Aspect 10 to further include the step of exposing the nematode-encapsulated capsule to radiation.

A method according to Aspect 13 of the present invention for evaluating response of nematodes is configured to include the steps of: supplying a test substance to a part of a nematode assay plate; supplying, to a predetermined position, the nematode-encapsulated capsule according to any one of Aspects 1 to 8; discharging the nematodes from the nematode-encapsulated capsule; and evaluating response of the nematodes to the test substance.

A method according to Aspect 14 of the present invention for evaluating response of nematodes may be configured in the above Aspect 13 such that the step of evaluating response of the nematodes to the test substance includes the step of, after lapse of a predetermined time period, (a) counting the number of nematodes that are attracted to the part of the nematode assay plate or nematodes that have escaped from to the part or (b) measuring an element co-related with the number of the nematodes.

A method according to Aspect 15 of the present invention for evaluating response of nematodes may be configured in the above Aspect 13 or 14 such that the test substance is a test substance that emits an odorant or a test substance that has a flavor.

A cancer test method according to Aspect 16 of the present invention includes the steps of: evaluating, by the method according to any one of the above Aspects 13 to 15, response of nematodes to a biological sample collected from a subject; and determining, by the step of evaluating, a possibility that the subject has cancer.

A method according to Aspect 17 of the present invention for evaluating response of nematodes to temperature, includes the steps of: adjusting a part of a nematode assay plate to a target temperature; supplying, to a predetermined position, the nematode-encapsulated capsule according to any one of the above Aspects 1 to 8; discharging the nematodes from the nematode-encapsulated capsule; and after lapse of a predetermined time period, (a) counting the number of nematodes that are attracted to the part of the nematode assay plate or nematodes that have escaped from the part or (b) measuring an element co-related with the number of the nematodes.

A nematode response evaluation kit according to Aspect 18 of the present invention includes the nematode-encapsulated capsule according to any one of the above Aspects 1 to 8.

A nematode response evaluation kit according to Aspect 19 of the present invention may be configured in the above Aspect 18 to be for a cancer test.

A production kit according to Aspect 20 of the present invention for producing a nematode-encapsulated capsule is a production kit for producing the nematode-encapsulated capsule according to any one of the above Aspects 1 to 4, the production kit including the nematode, the water-soluble polymer, and a salt of the cation.

A production kit according to Aspect 21 of the present invention for producing a nematode-encapsulated capsule is a production kit for producing the nematode-encapsulated capsule according to the above Aspect 5, the production kit including the nematode having been exposed to radiation, the water-soluble polymer, and a salt of the cation.

A production kit according to Aspect 22 of the present invention for producing the nematode-encapsulated capsule is a production kit for producing the nematode-encapsulated capsule according to Above Aspect 6, the production kit including the nematode having been treated with the hatching inhibitor, the water-soluble polymer, and a salt of the cation.

A production kit according to Aspect 23 of the present invention for producing a nematode-encapsulated capsule is a production kit for producing the nematode-encapsulated capsule according to the above Aspect 7, the production kit including the nematode having been subjected to a sterilization treatment, the water-soluble polymer, and a salt of the cation.

A production kit according to Aspect 24 of the present invention for producing a nematode-encapsulated capsule is a production kit for producing the nematode-encapsulated capsule according to the above Aspect 8, the production kit including the nematode, the water-soluble polymer, and a salt of the cation, and the hatching inhibitor.

The following will provide Examples to more specifically describe embodiments of the present invention. As a matter of course, the present invention is not limited to Examples provided below, but details of the present invention can be realized in various manners. Further, the present invention is not limited to the embodiments described above, and it may be varied in various ways within the scope of the appended Claims. Further, the technical scope of the present invention also encompasses an embodiment achieved by appropriately combining technical means separately disclosed. In addition, the content of all the literatures referred to in the present specification are incorporated herein by reference in their entirety.

Examples

In Examples and Comparative Examples shown below, hermaphrodites of *C. elegans* were used as nematodes. *C. elegans* was cultured and raised at 20°C, which was an optimal temperature. Table 1 shows the following conditions, among production conditions of each of differing nematode-encapsulated capsules of respective Examples: a developmental stage of nematodes to be encapsulated; whether or not a sterilization treatment is carried out in advance; a method of collecting nematodes and a method of adding the nematodes to aqueous sol; a type of buffer solution etc. to be used as a nematode wash solution and a nematode liquid; and the presence/absence of food in the nematode-encapsulated capsule.

[Table 1]

| Example | Nematodes to be encapsulated (main) | Sterilization treatment | Method of collecting/ adding nematodes | Type of nematode liquid | Food in capsule |
|---------|-------------------------------------|-------------------------|----------------------------------------|-------------------------|-----------------|
| 1 | adult (third day after hatching) | none | nematode liquid | Wash buffer | absent |
| 2 | egg | none | nematode liquid | Wash buffer | absent |

(continued)

| Example | Nematodes to be encapsulated (main) | Sterilization treatment | Method of collecting/ adding nematodes | Type of nematode liquid | Food in capsule |
|---|---|---|---|---|---|
| 3 | egg | none | nematode liquid | Wash buffer | present |
|  | egg | none | nematode liquid | M9 buffer | present |
| 4 | adult (third day after hatching) | none | nematode liquid | M9 buffer | present |
| 5 | adult (third day after hatching) | exposure to radiation | nematode liquid | M9 buffer | present |
| 6 | adult (fourth day after hatching) | exposure to hatching inhibitor | platinum wire picker | - | present |
|  | adult (fourth day after hatching) | none | platinum wire picker | - | present |
|  | adult (fourth day after hatching) | exposure to hatching inhibitor | nematode liquid | M9 buffer | present |
| 7 | adult (third day after hatching) | none | nematode liquid | Wash buffer | present |
| 8 | adult (third day after hatching) | none | nematode liquid | M9 buffer | present |
| 9 | adult (third day after hatching) | none | nematode liquid | Wash buffer | present |
| 10 | egg | none | nematode liquid | Wash buffer | present |

The following will describe Examples each related to the step of producing a nematode-encapsulated capsule and to culture of nematodes in the capsule.

[Example 1. Production of capsule in which nematode individuals (adults) were encapsulated]

A typical method for producing a nematode-encapsulated capsule according to an aspect of the present invention includes dropping and gelatinizing, in an aqueous solution of a calcium salt, an aqueous sol of a calcium-coagulable polymer compound in which nematodes are encapsulated. In the present Example, this method was used to produce an adult nematode-encapsulated capsule. For washing the nematodes, wash buffer was used. Further, as a nematode liquid to be used for injection of nematodes into the aqueous sol, wash buffer at 20°C containing washed nematodes (eggs or individuals) was used. The wash buffer was prepared by performing autoclaved sterilization of a liquid obtained by dissolving 0.5 g of gelatin powder in 993 mL of ultrapure water, and thereafter adding, to the liquid, 1 M of $MgSO_4$ (1 mL), 1 M of potassium phosphate (pH 6.0, 5mL), and 1 M of $CaCl_2$ (1 mL) and stirring a resultant mixture. To the nematode-encapsulated capsule produced in the present Example, *E. coli* that would be food was not added.

(Preparation of aqueous sol and aqueous solution of calcium salt)

First, sodium alginate (2 g) was dissolved into 200 mL of S-basal buffer which was generally used in culture or various assays of nematodes. Then, the aqueous sol that served as a raw material of the nematode-encapsulated capsule was prepared by stirring. The S-basal buffer was prepared by (i) adding, to a liquid obtained by dissolving NaCl (5.9 g) in 949 mL of ultrapure water, ethanol (1 mL) in which cholesterol (5 mg) was dissolved, and performing autoclaved sterilization of a resultant liquid, and (ii) thereafter adding 1 M potassium phosphate (pH 6.0, 50 mL) and performing stirring. Next, an aqueous solution of a calcium salt to be used in the production step of gelatinizing the aqueous sol was prepared by dissolving calcium lactate (2 g) in 200 mL of ultrapure water and performing stirring.

(Production of adult nematode-encapsulated capsule)

First, the wash buffer containing adult nematodes on the third day after hatching was dropped in the above-described aqueous sol, so that an aqueous sol containing the nematodes (which, hereinafter, may also be referred to as "nematode sol") was prepared. Next, a micropipette or an electric pipettor was used to suck out the nematode sol. The nematode sol was then dropped in a calcium lactate solution which filled a beaker, and immersed in the calcium lactate solution for approximately 5 minutes. The adult nematode-encapsulated capsule in which the adult nematodes were encapsulated was thus produced. At the end, the adult nematode-encapsulated capsule was taken out from the calcium lactate solution and an outer phase membrane of the adult nematode-encapsulated capsule was washed with ultrapure water.

(Result of Example 1)

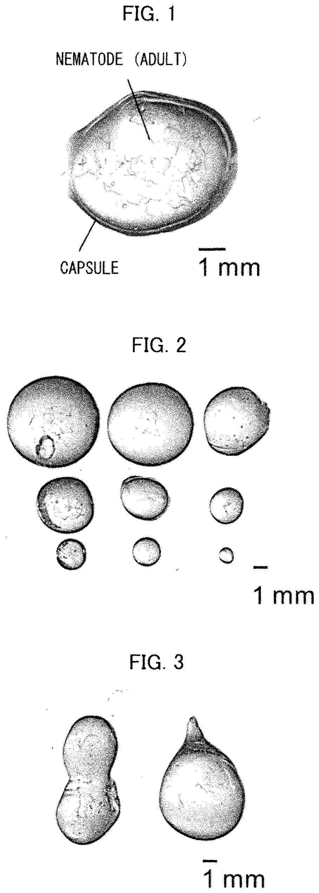

Fig. 1 is an image obtained by capturing from above an image of a spherical capsule in which approximately 100 adult nematodes were encapsulated, with use of a digital camera attached to a microscope. Further, Fig. 2 shows examples of spherical (adult) nematode-encapsulated capsules formed to have various sizes by adjusting (i) an amount of the nematode sol dropped and (ii) a tip diameter of the electric pipettor or a chip diameter of the micropipette. Further, Fig. 3 shows examples of (adult) nematode-encapsulated capsules formed into arbitrary shapes by, for example, reducing a liquid amount of the calcium lactate aqueous solution or setting a dropping position of the nematode sol immediately above a liquid surface of the calcium lactate aqueous solution.

It is apparent from Fig. 1 that adult nematodes were encapsulated in the capsule. Further, it is clear from Fig. 2 that the nematode-encapsulated capsules having various sizes can be freely produced by, for example, adjusting the amount of the nematode sol dropped. Further, it is clear from Fig. 3 that nematode-encapsulated capsules having arbitrary shapes such as a tear drop shape or a gourd shape other than a spherical shape can be produced under control or contingently.

It is clear from the above that a nematode-encapsulated capsule in which nematodes are encapsulated can be produced by dropping and immersing, in a calcium salt solution, a nematode sol obtained by injecting a nematode liquid into an aqueous sol.

[Example 2. Production of capsule in which nematode eggs were encapsulated and culture of nematode under low nutrition]

A nematode egg-encapsulated capsule in which eggs were encapsulated was produced by dropping and gelatinizing, in an aqueous solution of a calcium salt, an aqueous sol of a calcium-coagulable polymer compound in which nematode eggs were encapsulated. As a nematode liquid to be used for injection of nematodes into the aqueous sol, wash buffer at 20°C containing nematodes (eggs or individuals) which had been washed with wash buffer was used. To the nematode egg-encapsulated capsule produced in the present Example, *E. coli* that would be food was not added. Then, a survival state in the nematode egg-encapsulated capsule in a low-nutrient state was observed over time.

(Production of nematode egg-encapsulated capsule)

First, eggs of adult nematodes were collected by a conventionally-known method. Next, an egg-containing wash buffer was dropped in an aqueous sol that was the same as that prepared in Example 1. Then, an aqueous sol containing nematode eggs (which, hereinafter, may also be referred to as "egg sol") was prepared. Next, a micropipette was used to suck out the nematode sol. The nematode sol was then dropped in a calcium lactate solution which filled a beaker, and immersed in the calcium lactate solution for approximately 5 minutes. The nematode egg-encapsulated capsule in which the nematode eggs were encapsulated was thus produced. At the end, the nematode egg-encapsulated capsule was taken out from the calcium lactate solution and an outer phase membrane of the capsule was washed with ultrapure water.

(Result of Example 2)

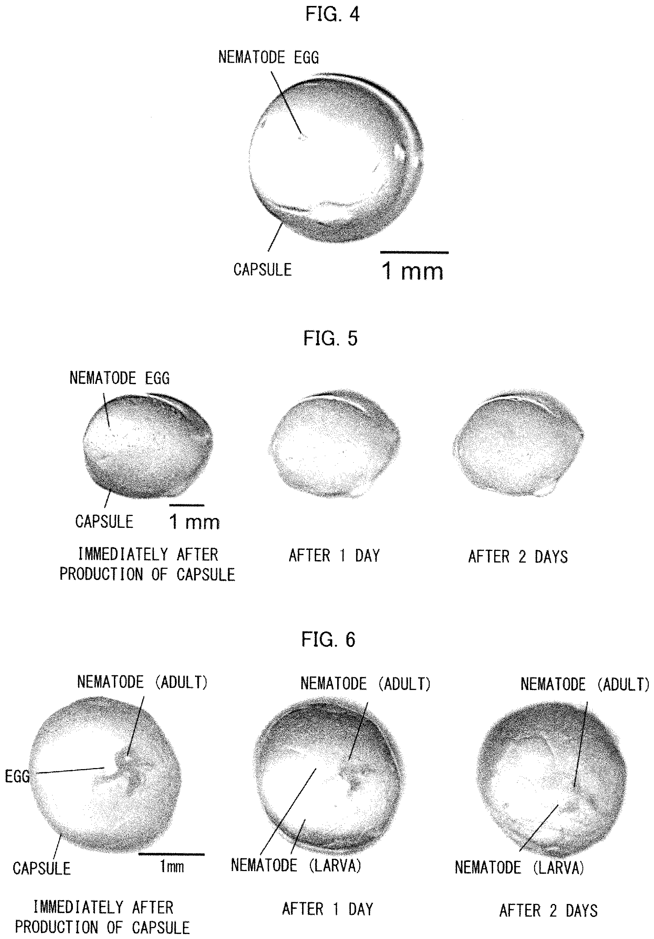

Fig. 4 is an image obtained by capturing from above an image of a spherical capsule in which approximately 10 adult nematode eggs were encapsulated, with use of a digital camera attached to a microscope. It is apparent from Fig. 4 that nematode eggs were encapsulated in the capsule. It is clear from the result of Example 1 that the nematode-encapsulated capsule can be produced by the same method for both eggs and individuals. Note that although not shown in the drawings, the nematodes in the capsule hatched into L1 larvae after one day. However, in the present Example in which *E. coli* that would be food was not added to the nematode egg sol, nutrition for growth was lacking. On this account, the nematodes were turned to be dauer larvae for once stopping the developmental stage in a starving state. Then, the larvae survived in the capsule for at least one month. This has revealed that by adjusting the nutritional state in the capsule, the nematodes can be positively maintained in the state of the dauer larvae for a long time.

[Example 3. Production of capsule in which nematode eggs were encapsulated and culture of nematodes in presence of *E. coli* that would be food]

A nematode-encapsulated capsule in which eggs were encapsulated was produced as in Example 2 by dropping and gelatinizing, in an aqueous solution of a calcium salt, an aqueous sol of a calcium-coagulable polymer compound in which nematode eggs were encapsulated. Further, as a nematode liquid to be used for injection of nematodes into the aqueous sol, wash buffer at 20°C containing nematodes (eggs or individuals) which had been washed with wash buffer was used. To the nematode egg-encapsulated capsule produced here, *E. coli* that would be food was added. Then, a survival state of the nematodes in the nematode egg-encapsulated capsule in a rich-nutrient state was observed over time.

(Production of nematode egg-encapsulated capsule)

As in Example 2, eggs were collected from adult nematodes on the third day after hatching, and an egg-containing wash buffer was dropped in an aqueous sol that was the same as that prepared in Example 1, so that an egg sol of the nematodes was prepared. Next, a suspension of *E. coli* that would be food was added to the egg sol. Further, a micropipette was used to suck out the nematode sol. The nematode sol was then dropped in a calcium lactate solution which filled a beaker, and immersed in the calcium lactate solution for approximately 5 minutes. The nematode egg-encapsulated capsule in which the nematode eggs and *E. coli,* which would be food, were encapsulated was thus produced. At the end, the nematode egg-encapsulated capsule was taken out from the calcium lactate solution and an outer phase membrane of the capsule was washed with ultrapure water.

(Result of Example 3)

An image of a spherical capsule in which approximately 100 nematode eggs were encapsulated was captured from above with use of a digital camera attached to a microscope and the spherical capsule was observed, every 24 hours (every day) from immediately after production of the spherical capsule. Fig. 5 shows results from immediately after the production up to two days later.
It is apparent from Fig. 5 that immediately after the production, the nematode eggs were encapsulated in the nematode-encapsulated capsule. In addition, after one day, the nematode eggs hatched into larvae (L1) in the capsule, and after two days, the larvae grew gradually. Further, although not shown in the drawings, the following were observed: after three days, the nematodes became adult nematodes; and after four days, laying eggs of a next generation was started. In contrast to the result of Example 2 in which the nematodes became dauer larvae due to the low-nutrient state, in the present example, growth at a normal rate was observed. This has revealed that it is possible to appropriately grow (culture) the nematodes by adjusting the nutritional state in the egg capsule.
Further, although not shown in the drawings, the nematode egg-encapsulated capsule was produced with use of M9 buffer, which is generally used in culture or various assays of nematodes, in place of the S-basal buffer in Example 3, and observation was made for several days. As a result, it was confirmed that the nematodes grew well from eggs to adult nematodes even when the M9 buffer was used as the solution. The M9 buffer was obtained as follows: a liquid (approximately 100 mL) prepared by dissolving NaCl (50 g), $KH_2PO_4$ (30 g), and $Na_2HPO_4$ (60 g) in ultrapure water was subjected to autoclaved sterilization; then 1 M of $MgSO_4$ (1 mL) was added and stirring was carried out; to a resultant solution, ultrapure water having been sterilized was added, so that the solution was diluted 10-fold; and then, stirring was carried out, so that the M9 buffer was prepared.

[Example 4. Production of capsule in which adult nematodes were encapsulated and culture of nematodes in presence of *E. coli* that would be food]

An adult nematode-encapsulated capsule was produced by dropping and gelatinizing, in an aqueous solution of a calcium salt, an aqueous sol which was made of a calcium-coagulable polymer compound and in which adult nematodes on the third day after hatching were encapsulated. As a nematode liquid to be used for injection of the nematodes into the aqueous sol, M9 buffer at 20°C was used which contained (adult) nematodes that had been collected from a culture plate with use of the M9 buffer.

(Production of adult nematode-encapsulated capsule)

Eggs were collected from adult nematodes and, until the third day after hatching, culturing was carried out on a plate to which *E. coli* was applied. Adult nematodes on the third day after hatching were collected from the culture plate with use of M9 buffer, and the M9 buffer was dropped in the aqueous sol which was the same as that prepared in Example 1, so that an adult nematode sol was prepared. Next, a micropipette was used to suck out the nematode sol. The nematode sol was then dropped in a calcium lactate solution which filled a plastic dish, and immersed in the calcium lactate solution for approximately 5 minutes. An adult nematode-encapsulated capsule in which the adult nematodes and *E. coli,* which would be food, were encapsulated was thus produced. At the end, the adult nematode-encapsulated capsule was taken out from the calcium lactate solution and an outer phase membrane of the adult nematode-encapsulated capsule was washed with ultrapure water.

(Result of Example 4)

An image of a spherical capsule in which approximately 10 adult nematodes on the third day after hatching were encapsulated was captured from above with use of a digital camera attached to a microscope and the spherical capsule was observed, every 24 hours (every day) from immediately after production of the spherical capsule. Fig. 6 shows results from immediately after the production up to two days later. It is apparent from Fig. 6 that from immediately after production of the adult nematode-encapsulated capsule, the adult nematodes in the capsule started to lay eggs. In addition, after one day, the nematode eggs hatched in the capsule, and not less than 100 larvae were observed. In addition, after two days, the number of larvae increased further, and the capsule was filled with larvae. The hatching rate of the eggs laid by the adult nematodes in the capsule was not different from that in the case of typical culture on a medium, and normal development was observed. The above result has revealed that egg-laying of adult nematodes and hatching of eggs in the capsule are possible.

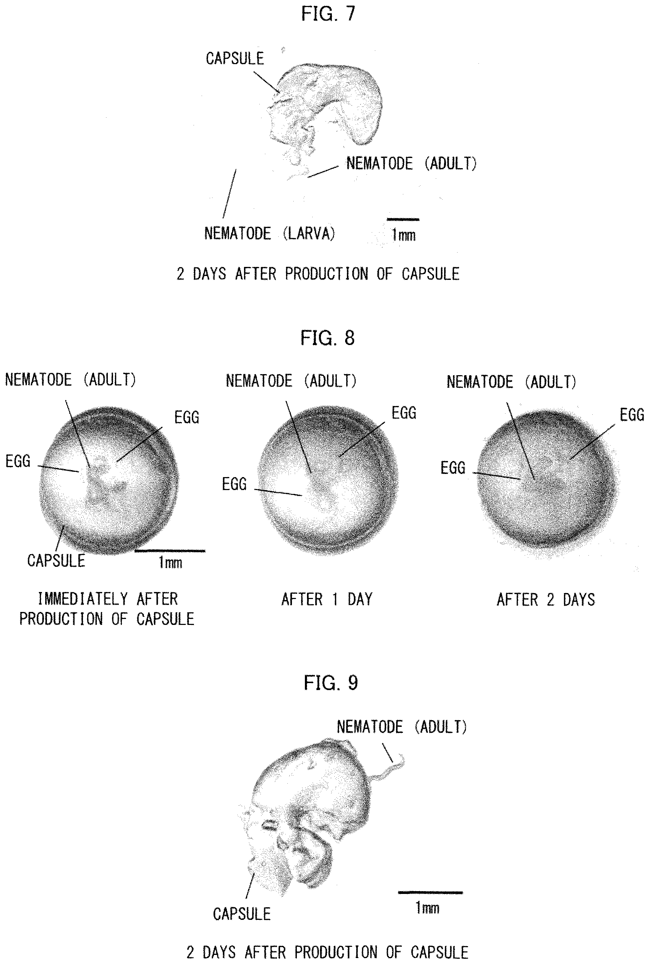

Next, the nematodes having been grown in the nematode-encapsulated capsule were taken out, and locomotion of these nematodes was observed. A commercially-available circular plastic dish which had a bottom surface having an inner diameter of 8.4 cm was used as a container for the nematode assay plate. Into the container, 10 mL of a solid medium mainly made of agar was poured, and was used as a plate A1 (described later). A nematode-encapsulated capsule after two days from production of the capsule was transferred to this plate A1 with a tweezer, and the capsule was picked a plurality of times with a sterile toothpick, so that the inside of the capsule was exposed. Fig. 7 shows a state in which the nematodes crawled out onto the plate A1 immediately after this exposure of the inside of the capsule. From Fig. 7, it was observed that the eggs, which had been laid in the capsule by the adult nematodes (parents) encapsulated in the production of the nematode-encapsulated capsule, hatched into larvae and the larvae crawled out of the capsule and moved normally on the plate A1. Further, it has been also clarified that, in a case where the number of adult nematodes (parents) in a nematode-encapsulated capsule is large relative to the size of the capsule, a large number of next-generation nematodes (offspring) may occur and a parental generation may be debilitated or die out.

Although not shown in the drawings, in a case where the nematode-encapsulated capsule was picked several times with a sterile toothpick one day after production and the inside of the capsule was exposed, the adult nematodes (parents) encapsulated in the capsule crawled out together with larvae of the next generation. This result has revealed that, if most nematodes of the next generation are within one day after egg-laying, that is, in a stage from eggs to L1 larvae, the adult nematodes can also be maintained normally.

In light of the above, in an experiment in which the parental generation of nematodes encapsulated in a nematode-encapsulated capsule is to be taken out of the capsule and used, it is effective to adjust capsule production conditions, culture conditions, and the like (e.g., the number of individuals to be encapsulated in the capsule or the size of the capsule, and the number of days of encapsulation) in order to allow the parental generation to survive together with the next generation. Alternatively, a method for inhibiting hatching of eggs, which will be described later, can be selected.

[Example 5. Production of capsule in which adult nematodes having been exposed to radiation were encapsulated and culture of nematodes in presence of *E. coli* that would be food]

An experiment was conducted to confirm effectiveness of a nematode-encapsulated capsule in which nematodes having been sterilized by exposure to radiation were encapsulated. An aqueous sol of a calcium-coagulable polymer compound in which adult nematodes that were on the third day after hatching and that had been exposed to cobalt-60 ($^{60}$Co) gamma rays were encapsulated was dropped in an aqueous solution of a calcium salt and gelatinized. An irradiated-nematode-encapsulated capsule was thus produced. The nematodes to be used for injection into the aqueous sol were adult nematodes having been exposed to cobalt 60 gamma rays while swimming in a tube containing M9 buffer, and a nematode liquid immediately after nematode irradiation was used.

(Exposure of nematodes to radiation)

As in Example 4, eggs were collected from adult nematodes and, until the third day after hatching, culturing was carried out on a plate to which *E. coli* was applied. Adult nematodes on the third day after hatching were collected from a culture plate with use of M9 buffer. A suspended nematode liquid in a tube was exposed to 500 Gy of cobalt 60 gamma rays, which was equivalent to a sterilization dose for hermaphrodites.

(Production of adult nematode-encapsulated capsule having been exposed to radiation)

The nematode liquid having been exposed to the gamma rays was dropped, immediately after irradiation, in the aqueous sol which was the same as that prepared in Example 1, so that a sterilized adult nematode sol was prepared. Next, a micropipette was used to suck out the nematode sol. The nematode sol was then dropped in a calcium lactate solution which filled a plastic dish, and immersed in the calcium lactate solution for approximately 5 minutes. An irradiated-nematode-encapsulated capsule in which adult nematodes exposed to radiation and *E. coli,* which would be food, were encapsulated was thus produced. At the end, a sterilized-nematode-encapsulated capsule was taken out from the calcium lactate solution and an outer phase membrane of this capsule was washed with ultrapure water.

(Result of Example 5)

An image of a spherical capsule in which approximately 10 adult nematodes on the third day after hatching and immediately after exposure to gamma rays were encapsulated was captured from above with use of a digital camera attached to a microscope and the spherical capsule was observed, every 24 hours (every day) from immediately after production of the spherical capsule. Fig. 8 shows results from immediately after the production up to two days later. As shown in Fig. 8, it is apparent that from immediately after production of the nematode-encapsulated capsule, the adult nematodes in the capsule started to lay eggs. After one day and also after two days, it was possible to observe a state in which the adult nematodes moved inside the capsule. However, the eggs laid by the adult nematodes did not hatch, and no larvae were observed.

All conditions and procedures in Example 5 were the same as those in Example 4, except that adult nematodes were exposed to gamma rays prior to production of the nematode-encapsulated capsule. In view of the result of Example 4, it is clear that by subjecting the nematodes to a sterilization (hatching inhibition) treatment through exposure to radiation, development of the next generation is inhibited even in a case where adult nematodes on the third day after hatching that start laying eggs are encapsulated, and thus it is possible to maintain a state immediately after production of the capsule in which only a parental generation is present.

Next, the nematodes having been grown in the nematode-encapsulated capsule were taken out, and locomotion of the nematodes was observed. As in Example 4, an irradiated-nematode-encapsulated capsule obtained two days after production of the capsule was transferred to a plate A1 with a tweezer, and the capsule was picked a plurality of times with a sterile toothpick, so that the inside of the capsule was exposed. Fig. 9 shows a state in which the nematodes crawled out onto a surface of the plate A1 immediately after the above exposure of the inside of the capsule. It is conventionally known that the locomotion of the nematodes having been exposed to 500 Gy of cobalt 60 gamma rays temporarily declines but the locomotion recovers after one day. Also in the present Example, no remarkable abnormality in the locomotion was observed in the case of the adult nematodes (parents) which were encapsulated in the capsule immediately after exposure to radiation and which were caused to escape from the nematode-encapsulated capsule after two days. It is apparent from this result that even in a case where adult nematodes having been exposed to a radiation dose equivalent to a sterilization dose were encapsulated in a capsule for approximately two days, no problem occurs in motor function.

[Example 6. Production of capsule in which adult nematodes having been exposed to hatching inhibitor were encapsulated and culture of nematodes in presence of *E. coli* that would be food]

A hatching inhibitor-treated nematode-encapsulated capsule was produced by dropping and gelatinizing, in an aqueous solution of a calcium salt, an aqueous sol which was made of a calcium-coagulable polymer compound and in which adult nematodes on the fourth day after hatching were encapsulated. In the present Example, no nematode liquid was used, and the adult nematodes were collected one at a time from a culture plate with use of a platinum wire picker, and then the adult nematodes were transferred into the aqueous sol together with *E. coli.*

(Hatching inhibitor treatment on nematodes)

The nematodes were cultured on the plate to which *E. coli* had been applied, until the nematodes became adult nematodes (before start of egg laying) on the third day after hatching. FUdR solution was added to this nematode culture plate, and the nematodes were cultured for another day. The FUdR solution was prepared by dissolving 5 mg of FUdR powder in 10 mL of sterile ultrapure water and stirring. It was confirmed that, although the adult nematodes laid eggs to a certain extent on the culture plate to which FUdR was added, the eggs did not hatch.

(Production of adult nematode-encapsulated capsule having been exposed to hatching inhibitor)

Adult nematodes on the fourth day after hatching which had been exposed to a hatching inhibitor for one day were collected together with *E. coli* from the culture plate with use of the platinum wire picker. Then, the nematodes were dropped in the aqueous sol which was the same as that prepared in Example 1, so that a sterilized adult nematode sol was prepared. Next, a micropipette was used to suck out the nematode sol. The nematode sol was then dropped in a calcium lactate solution which filled a plastic dish, and immersed in the calcium lactate solution for approximately 5 minutes. The hatching inhibitor-treated nematode-encapsulated capsule in which the adult nematodes that had been exposed to the hatching inhibitor and *E. coli* that would be food were encapsulated. At the end, a sterilized-nematode-encapsulated capsule was taken out from the calcium lactate solution and an outer phase membrane of this capsule was washed with ultrapure water.

(Result of Example 6)

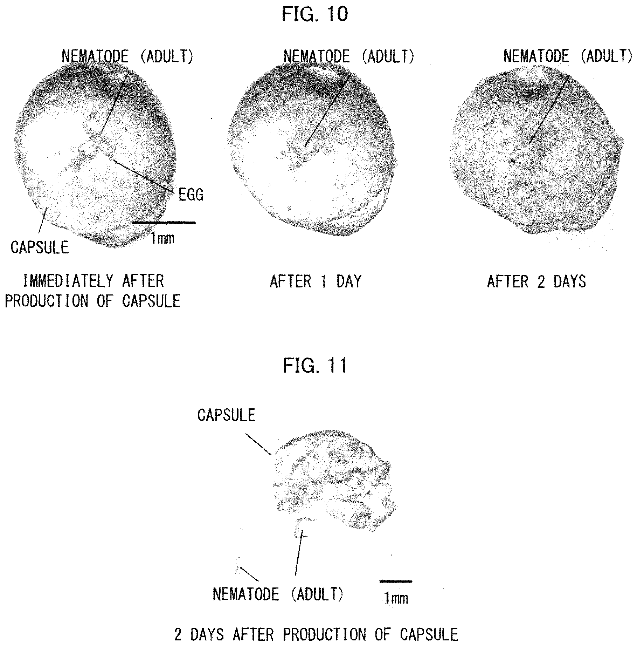

An image of a spherical capsule in which approximately 10 adult nematodes on the fourth day after hatching and having undergone a hatching inhibition treatment by exposure to FUdR were encapsulated was captured from above with use of a digital camera attached to a microscope and the spherical capsule was observed, every 24 hours (every day) from immediately after the production of the spherical capsule. Fig. 10 shows results from immediately after the production up to two days later. As shown in Fig. 10, it is apparent that from immediately after the production of the nematode-encapsulated capsule, the adult nematodes in the capsule started to lay eggs. After one day and also after two days, it was possible to observe a state in which the adult nematodes moved inside the capsule. However, the eggs laid by the adult nematodes did not hatch, and no larvae were observed.

Next, the nematodes having been grown in the nematode-encapsulated capsule were taken out, and locomotion of the nematodes was observed. As in Example 4, a hatching inhibitor-treated nematode-encapsulated capsule obtained two days after production of the capsule was transferred to a plate A1 with a tweezer, and the capsule was picked a plurality of times with a sterile toothpick, so that the inside of the capsule was exposed. Fig. 11 shows a state in which the nematodes crawled out onto a surface of the plate A1 immediately after the above exposure of the inside of the capsule. No remarkable abnormality in locomotion was observed in the case of the adult nematodes (parents) which were caused to escape from the nematode-encapsulated capsule two days after the production of the capsule. It is clear from this result that even in a case where adult nematodes having been exposed to the hatching inhibitor are encapsulated in a capsule for approximately two days, no problem occurs in motor function.

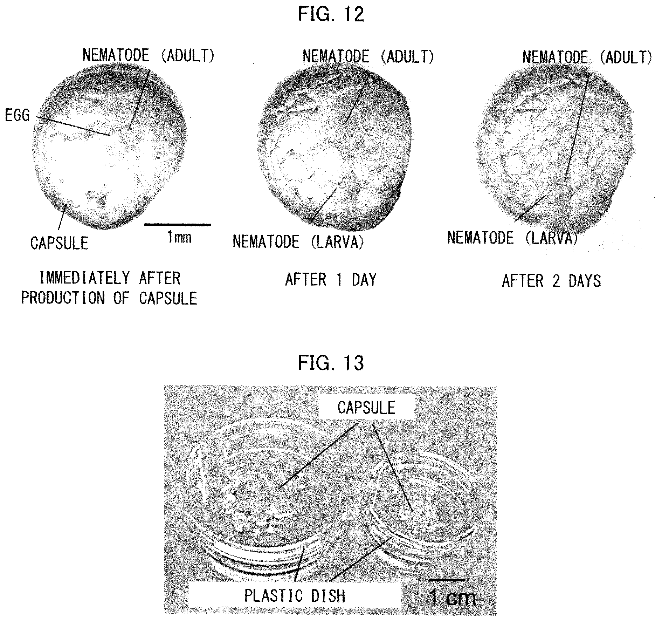

As shown in Figs. 10 and 11, it was confirmed that development of the next generation was inhibited in the capsule in which the adult nematodes (corresponding to an egg-laying stage) on the fourth day after hatching which had been subjected to the treatment of exposure to the hatching inhibitor were encapsulated. In addition, it was confirmed that it was possible to maintain, for at least two days, a state immediately after production of the capsule in which only a parental generation was present. In order to confirm that the absence of emergence of the next generation, except for the eggs, was an effect of the exposure to the hatching inhibitor, a nematode-encapsulated capsule was produced in a similar manner with use of nematodes cultured for one day on a culture plate to which M9 buffer, instead of the FUdR solution (M9 buffer containing FUdR), was added. Fig. 12 shows a result of such a control. As shown in Fig. 12, the adult nematodes laid eggs in the capsule and after 2 days, the capsule was filled with larvae that have hatched, as in the result of Example 4 in which the nematodes on the third day after hatching were used. This reveals that it was an effect of the treatment of exposure to the hatching inhibitor that in the capsule in which adult nematodes having been exposed to the hatching inhibitor were encapsulated, the adult nematodes laid eggs, but the eggs did not hatch and larvae did not appear.

Further, although not shown in the drawings, in Example 6 which will be described later, it was confirmed that, even in the case of a nematode-encapsulated capsule produced with use of nematodes cultured for one day in a culture solution containing FUdR in place of culturing for one day on a culture plate to which the FUdR solution was added, a result similar to that obtained by culturing on the culture plate to which the FUdR solution was added could be obtained.

It is clear, from the results of Examples 1 to 3 and 4 to 6 each related to a production step of the nematode-encapsulated capsule and nematode culture in the capsule, that the nematode-encapsulated capsule can be produced even in a case where conditions related to the production of the capsule and the nematode culture are variously changed. Examples of such conditions encompass: a developmental stage of nematodes to be encapsulated in the capsule; whether or not to carry out the sterilization treatment; a method of collecting nematodes from a culture plate; a method for adding nematodes to an aqueous sol; a type of nematode liquid to be used for washing or culturing nematodes; and whether or not to add food in the capsule. Further, it has been confirmed that by adjusting, according to an intended use of the nematodes, the production conditions of the nematode-encapsulated capsule or the conditions for the nematode culture, it is possible to inhibit development of the next generation or control the nutritional state of the next generation, to control the number of individuals, and/or the like.

The following will describe Examples related to storage of nematode-encapsulated capsules and biodegradation of nematode-encapsulated capsules in soil.

[Example 7. Storage of nematode-encapsulated capsules]

(Production of adult nematode-encapsulated capsules)

The nematode sol was prepared by the method described in Example 1, and a suspension of *E. coli* that would be food was added. Next, a micropipette was used to suck out the nematode sol. The nematode sol was then dropped in a calcium lactate solution which filled a beaker, and immersed in the calcium lactate solution for approximately 5 minutes. Thus, spherical nematode-encapsulated capsules which had a diameter of approximately 3 mm and in which adult nematodes were encapsulated were produced. Further, an electric pipettor was used to suck out the nematode sol. Then, the nematode sol was dropped in a calcium lactate solution which filled a beaker, and immersed in the calcium lactate solution for approximately 5 minutes. Thus, spherical nematode-encapsulated capsules which had a diameter of approximately 5 mm and in which adult nematodes were contained were produced. At the end, the adult nematode-encapsulated capsules were taken out from the calcium lactate solution and an outer phase membrane of each of the nematode-encapsulated capsules was washed with ultrapure water.

(Storage of nematode-encapsulated capsules)

Approximately 30 spherical nematode-encapsulated capsules which had a diameter of approximately 3 mm and which had been washed were transferred to a plastic dish which had a bottom surface having a diameter of approximately 2.8 cm. In addition, approximately 30 spherical nematode-encapsulated capsules which had a diameter of approximately 5 mm and which had been washed were transferred to a plastic dish which had a bottom surface having a diameter of 5.4 cm. Those plastic dishes were each covered with a lid and sealed with a paraffin film, after a liquid component was wiped out with a paper wiper.

(Result of Example 7)

Fig. 13 shows respective examples of the above two plastic dishes in which spherical nematode-encapsulated capsules having two different sizes were stored. Note that, in practice, the plastic dishes are sealed with the paraffin film and stored, but in order that the content of the plastic dishes can be seen well, Fig. 13 shows the plastic dishes on which the paraffin film had not yet been wrapped. The nematodes in the nematode-encapsulated capsules on the plastic dishes which had been sealed with the paraffin film and which then were stored remained alive in a good condition even after three days. It is clear from this that the method for storing according to the present Example does not adversely affect survival of the nematodes in the nematode-encapsulated capsules.

Further, it has been revealed that by increasing the number of nematode-encapsulated capsules to be stored in a single plastic dish from Example shown in Fig. 13 and storing the nematode-encapsulated capsules in a dense condition, it is possible to inhibit rolling of the capsules due to vibration during transport.

Further, as another method, it is possible to reduce the effect of vibration on the nematodes during transport by making at least one surface of each of the nematode-encapsulated capsule flat or by filling a gap with liquid.

[Example 8. Biodegradation of nematode-encapsulated capsules in soil]

It may be possible to gradually reduce the number of individuals of pest nematodes, by (i) sterilizing pest nematodes that cause great damage to agricultural crops or (ii) sterilizing antagonistic nematodes and returning those antagonistic nematodes to soil. By encapsulating intended nematodes in capsules and scattering the capsules, it is possible to expect the effect of, for example, improving work efficiency and/or slowing down the rate at which the nematodes are transferred to the soil. In the present Example, in order to evaluate environmental affinity of the nematode-encapsulated capsules, whether or not the capsules could be biodegraded in the soil was investigated.

(Soil affinity evaluation test of nematode-encapsulated capsules)

Spherical nematode-encapsulated capsules having a diameter of 2 mm to 3 mm were produced by the method according to Example 4. In the nematode-encapsulated capsules, adult nematodes on the third day after hatching were encapsulated. In a commercially-available circular plastic dish which had a bottom surface having a diameter of 5.4 cm, commercially-available leaf mold was bedded and moistened with water. Thereafter, approximately 50 nematode-encapsulated capsules were scattered. Then, the plastic dish was covered with a lid and left to stand at 20°C.

(Result of Example 8)

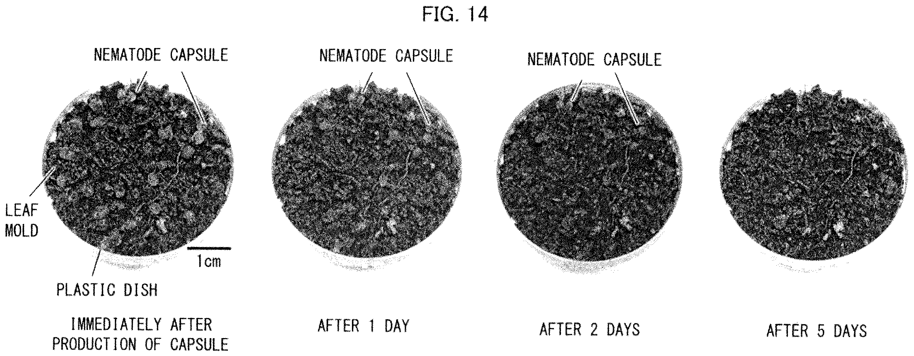

A state of the nematode-encapsulated capsules on the leaf mold spread on the plastic dish was observed by opening the lid every 24 hours (every day) from immediately after the nematode-encapsulated capsules were scattered. Fig. 14 shows results obtained immediately after the scattering, after one day, after two days, and after five days. As shown in Fig. 14, the nematode-encapsulated capsules scattered on the leaf mold immediately after the production (encapsulation of the nematodes) were gradually assimilated to the leaf mold after one day and after two days. Five days later, biodegradation of the outer phase membranes proceeded to a degree that the shape of the nematode-encapsulated capsules could not be visually observed. When a part of the leaf mold was taken out and observed with use of a microscope, it was possible to observe a state in which a plurality of nematodes moved around in a mixture of fragments of the nematode-encapsulated capsules and the leaf mold.

From the above result, it can be said that a nematode-encapsulated capsule is a simple and efficient tool for transferring a desired nematode to soil. Since the capsule is made a highly biodegradable material, the capsule does not become an environmental load. Thus, it is clear that the capsule is highly useful in the fields of agriculture, forestry, and fisheries.

The following will describe Examples and Comparative Examples each related to the supply step of nematodes and the nematode response evaluation assay. In each of the Examples and Comparative Examples, a commercially-available circular plastic dish which had a bottom surface having an inner diameter of 8.4 cm was used as a container for the nematode assay plate. Into the container, 10 mL of a solid medium mainly made of agar was poured, and was used as a plate A1. In addition, also used were a plate A2 in which one recessed part, which served as the nematode supply part, was made by hollowing out the solid medium of the plate A1 and a plate A3 in which four recessed parts, which served as nematode fall-in cavities, were formed by hollowing out two left parts and two right parts of the solid medium of the plate A1. Note that the recessed part which served as the nematode supply part was a cylindrical recess which had an inner diameter of 5 mm and the center of which was the center of the solid medium, and the recessed parts which served as the nematode fall-in cavities were each a cylindrical recess which had an inner diameter of 5 mm in the solid medium. Table 2 shows details of the nematode assay plate used in Examples. The plate A3 is identical to a plate A1 disclosed in Patent Literature 2.

[Table 2]

| Name | Solid medium | | | | Container | | |
|---|---|---|---|---|---|---|---|
| | Amount of solid medium (mL) | Thickne ss (cm) | Number of supply parts | Number of fall-in cavities | Inner diameter of bottom surface (cm) | Bottom area (cm2) | Height inside container (cm) |
| Plate A1 | 10.0 | approximately 0.18 | 0 | 0 | approximately 8.4 | approximately 55.0 | 1.5 |
| Plate A2 | | | 1 | 0 | | | |
| Plate A3 | | | 0 | 2 left and 2 right cavities | | | |

[Example 9. Consideration of nematode supply step]

The nematode supply step with use of the nematode-encapsulated capsule according to an aspect of the present invention was evaluated with use of a plate A1 where 10 mL of a solid medium was poured and a plate A2 that was provided with one recessed part in the center into which the solid medium was similarly poured and which served as a supply part for supplying nematodes.

First, one capsule which were produced as in Example 1 and in which adult nematodes were encapsulated was pinched with a tweezer. Then, the capsule was left to stand still at each of supply parts on the plate A1 and the plate A2, that is, the center of the plate A1 and the recessed part in the center of the plate A2. In each of the plates, the nematode-encapsulated capsule was picked several times with a sterile toothpick, and the inside of the capsule was exposed. As a result, the nematodes crawled out to the periphery of the supply part of each of the plate A1 and the plate A2. A liquid component contained in the capsule was sucked with a micropipette. A state in which the nematodes dispersed on a surface of the solid medium was observed by capturing, from above with use of a digital camera attached to a microscope, an image of an approximately 1.4 cm square of each of the plate A1 and the plate A2 every minute from immediately after supply of the nematode-encapsulated capsule. In the approximately 1.4 cm square, the supply part of each of the plates A1 and A2 was located at the center. The state in which the nematodes dispersed on the surface of the solid medium was observed.

(Result of Example 9)

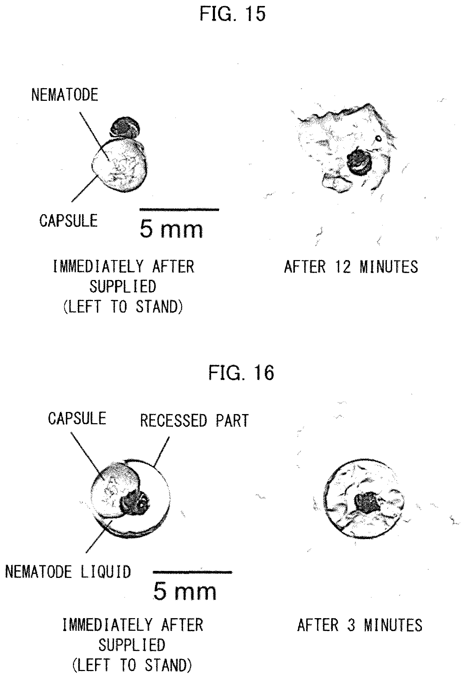

Figs. 15 and 16 show respective results of observation over time of the peripheries of the supply parts of the plate A1 and the plate A2. Note that each of Figs. 15 and 16 shows an image immediately after supply of the nematode-encapsulated capsule and an image at a time point when almost all nematodes had crawled out onto the plate in this order from the left. A black dot in the center of each of the images is a mark indicating the center of the supply part.

It was found, as shown in each of Figs. 15 and 16, that in a case where the nematode-encapsulated capsule was supplied, the nematodes crawled out within 12 minutes when the plate A1 or the plate A2 were used. Further, in the case of the plate A2, the nematodes crawled out within three minutes. This has revealed that use of a method in which nematodes are supplied to the recessed part (nematode supply step using a supply part-processed plate) in combination with the nematode-encapsulated capsule according to an aspect of the present invention increases efficiency of supply of the nematodes.

In addition, in an operation to pick and break the nematode-encapsulated capsule with a toothpick, the nematode-encapsulated capsule on the plate A2, on which the nematode-encapsulated capsule was left to stand on a bottom surface of the plate A2 where the recessed part was formed and exposed, was less likely to roll and thus easier to pick than that on the plate A1, on which the nematode-encapsulated capsule was left to stand on agar. Further, the plate A2 had the advantage that spread of a solution which contained the nematodes and which had flown out from the nematode-encapsulated capsule was restrained within the recessed part and that a point from which the nematodes start to move could be controlled within a certain range.

(Comparative Example 1. Supplying nematodes by conventional method)

The nematode supply step by a conventionally known method was evaluated with use of a plate A1 where 10 mL of the solid medium was poured and a plate A2 where the solid medium was similarly poured and the recessed part for supplying nematodes was provided in the center (nematode supply step using a supply part-processed plate).

First, a nematode liquid containing adult nematodes having been washed was sucked out with a micropipette, and a drop of the nematode liquid containing approximately 100 adult nematodes was dropped on the supply part of each nematode assay plate. A liquid component of the nematode liquid on the plate A1 was removed by a conventionally-known method, that is, by wiping out the liquid component with use of a paper wiper. A liquid component of the nematode liquid in the recessed part of the plate A2 was removed by a method in which the liquid component was sucked with use of a micropipette. A state in which the nematodes dispersed on a surface of the solid medium was observed by capturing, from above with use of a digital camera attached to a microscope, an image of an approximately 1.4 cm square of each of the plate A1 and the plate A2. In the approximately 1.4 cm square, the supply part of each of the plates A1 and A2 was located at the center.

(Result of Comparative Example 1)

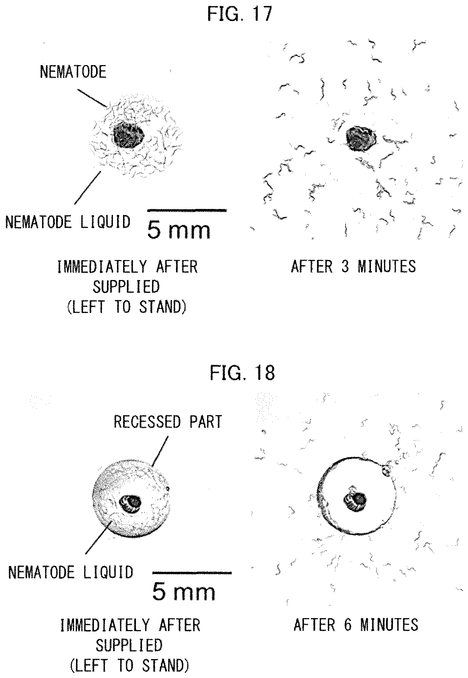

Figs. 17 and 18 show respective results of observation over time of the peripheries of the supply parts of the plate A1 and the plate A2. Note that each of the Figs. 17 and 18 shows an image immediately after supply of the nematode liquid and an image at a time point when almost all nematodes had crawled out onto the nematode assay plate in this order from the left. A black dot in the center of each of the images is a mark indicating the center of the supply part.

As shown in Figs. 17 and 18, in a case where the nematode liquid was supplied, dispersion of the nematodes completed within 6 minutes when the plate A1 and the plate A2 were used. In a comparison between a result of supplying the nematodes in Example 9 using the nematode-encapsulated capsule according to an aspect the present invention and a result of the present Comparative Example, no significant difference was found between those results. It has been thus found that the nematode-encapsulated capsule according to an aspect of the present invention can be used as a method alternative to a conventional nematode supply method.

[Example 10. Assay for evaluating response of nematodes to volatile substance with use of nematode-encapsulated capsule]

An assay for evaluating response of nematodes to a volatile substance was conducted with use of diacetyl (having a molarity of 11.5 M at 15°C), which is a kind of volatile substance, and diluted solutions prepared by diluting the diacetyl by two different dilution factors of 10 and $10^5$. Note that the assay method was a method using a nematode trap plate.

First, a plate A3 was prepared so as to be include (a) two left fall-in cavities that were formed in a left part of a solid medium of a plate A1 identical to that used in Example 1 so as to be arranged side by side along the outer periphery of the solid medium and (b) two right fall-in cavities formed in control positions in a right part of the solid medium in a similar manner to the left fall-in cavities. The left fall-in cavities were filled with a liquid to be tested (hereinafter, such a liquid may be referred to as "test solution"), whereas the right fall-in cavities were filled with a buffer solution that served as a control. As described above, the test solution used in the present Example included the diluted solutions of diacetyl, which is a kind of volatile substance. Note that for the above dilution, the buffer solution, which served as the control was used.

Next, several nematode-encapsulated capsules in which nematode eggs were encapsulated and grown to adult nematodes were each picked with a tip of a micropipette chip, so that an outer phase membrane was broken. Then, a solution containing nematodes was sucked out of the capsule, and dropped onto the center of the plate A3. Similarly, the solution containing nematodes sucked out of several of the same capsules was dropped in the same amount on another plate A3 for another test solution having a different concentration to be tested simultaneously. Thereafter, a liquid component of this solution was wiped out with a paper wiper, and the nematodes were released onto the plate. The plate was covered with a lid and sealed with a paraffin film. Then, the plate was left to stand at least for an hour under a light-shielded environment. Thereafter, the number of nematodes caught in the fall-in cavities was counted.

(Result of Example 10)

Assuming that the total number of nematodes caught in the two left fall-in cavities filled with the test solution was $N_1$ and the total number of nematodes caught in the two right fall-in cavities filled with the control buffer solution was $N_2$, a value obtained according to the following formula was defined as a chemotaxis index (C.I.). Then, the response was evaluated.

$$\text{Chemotaxis index (C. I.)} = (N_1 - N_2)/(N_1 + N_2).$$

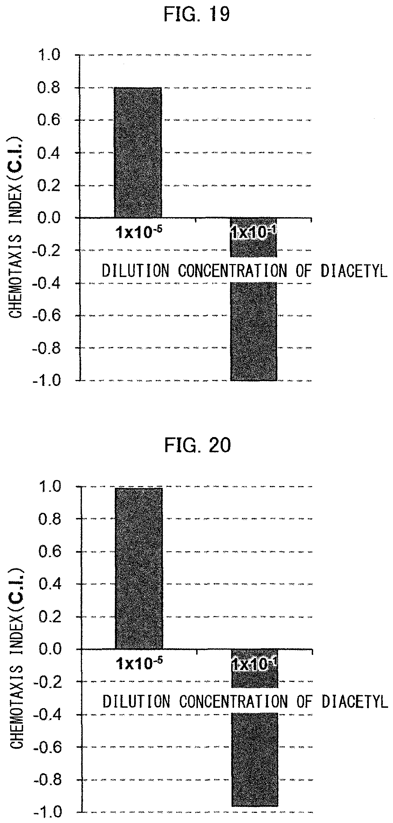

A positive value means that the nematodes were attracted to the left fall-in cavities, that is, to diacetyl, whereas a negative value means that the nematodes escaped from the left fall-in cavities. Note that assays were conducted with use of diluted solutions at three different concentrations, that is, the assays were conducted on test solutions of all of the three different concentrations. Fig. 19 shows mean values of C. I. obtained in two times of assays conducted independently.

As shown in Fig. 19, with a 10-fold diluted solution, the value was negative. This indicated that the nematodes escaped from diacetyl. In contrast, with a $10^5$-fold diluted solution, the value was positive. This indicated that the nematodes were attracted to diacetyl.

[Comparative Example 2. Assay for evaluating response of nematodes to volatile substance with use of conventional nematode supply method]

As Comparative Example, evaluation of response of nematodes was carried out on the basis of a conventionally-known method for supplying nematodes. Used was a plate A3 which was identical to that used in Example 10 and in which two sets of two fall-in cavities were formed on left and right sides in symmetrical positions. A test solution was diluted solutions of diacetyl at two different concentrations as in Example 10. Note that the above dilution was conducted with use of a buffer solution, which served as a control.

A nematode liquid containing nematodes having been washed in advance with wash buffer was dropped onto the center of the nematode assay plate, and a liquid component was wiped out with a paper wiper. Then, the nematodes were released on the plate. The plate was covered with a lid and sealed with a paraffin film. Then, the plate was left to stand at least for an hour under a light-shielded environment. Thereafter, the number of nematodes caught in the fall-in cavities was counted.

(Result of Comparative Example 2)

Fig. 20 shows mean values of C. I. obtained in three times of assays conducted independently, for the test solutions of all of three different concentrations. As shown in Fig. 20, with a 10-fold diluted solution, the value was negative. This indicated that the nematodes escaped from diacetyl. In contrast, with a $10^5$-fold diluted solution, the value was positive. This indicated that the nematodes were attracted to diacetyl.

The results of Example 10, in which the assay was conducted by the supply method with use of the nematode-encapsulated capsule agreed well with the results of Comparative Example 2, in which the assay was conducted by the conventional nematode supply method. This reveals that the nematodes encapsulated in the capsule at the egg stage and grown to adult nematodes can be used for conducting a response evaluation assay of nematodes without any problem.

Industrial Applicability

An aspect of the present invention solves potential problems related to preparation and supply of nematodes in fields of pest control, education, research, and industries (for example, microbial experiments, cell culture experiments, cancer tests using nematodes, and space experiments). It is expected that an aspect of the present invention will greatly contribute to improvement of efficiency and reduction of cost of various biological experiments.

**Claims**

1. A nematode-encapsulated capsule comprising:

   an encapsulation composition containing a nematode; and
   at least one layer made of membrane with which the encapsulation composition is encapsulated,
   the membrane containing, as a main component, a water-soluble polymer that gelatinizes by reacting with a cation.

2. The nematode-encapsulated capsule according to claim 1, having an outer diameter of not more than 10 cm.

3. The nematode-encapsulated capsule according to claim 1, wherein the nematode is *Caenorhabditis elegans* or a related species thereof.

4. The nematode-encapsulated capsule according to claim 1, wherein the water-soluble polymer is at least one kind of water-soluble polymer selected from the group consisting of proteins and polysaccharides.

5. The nematode-encapsulated capsule according to claim 1, wherein the nematode is a nematode having been exposed to radiation.

6. The nematode-encapsulated capsule according to claim 1, wherein the nematode is a nematode having been treated with a hatching inhibitor.

7. The nematode-encapsulated capsule according to claim 1, wherein the nematode is a nematode having been subjected to a sterilization treatment.

8. The nematode-encapsulated capsule according to claim 1, wherein the encapsulation composition further contains a hatching inhibitor.

9. A method for producing the nematode-encapsulated capsule according to any one of claims 1 to 8, the method comprising the steps of:

   preparing the encapsulation composition by mixing the nematode and the water-soluble polymer with each other; and
   producing the nematode-encapsulated capsule by reacting the encapsulation composition and a solution containing the cation.

10. A method for producing the nematode-encapsulated capsule according to any one of claims 1 to 8, the method comprising the steps of:

preparing a base material of the membrane of the nematode-encapsulated capsule by reacting a solution containing the water-soluble polymer and a solution containing the cation; and
producing the nematode-encapsulated capsule by injecting the nematode into the base material.

11. The method according to claim 9, further comprising the step of exposing the nematode-encapsulated capsule to radiation.

12. The method according to claim 10, further comprising the step of exposing the nematode-encapsulated capsule to radiation.

13. A method for evaluating response of nematodes, comprising the steps of:

supplying a test substance to a part of a nematode assay plate;
supplying, to a predetermined position, the nematode-encapsulated capsule according to any one of claims 1 to 8;
discharging the nematodes from the nematode-encapsulated capsule; and
evaluating response of the nematodes to the test substance.

14. The method according to claim 13, wherein the step of evaluating response of the nematodes to the test substance includes the step of, after lapse of a predetermined time period, (a) counting the number of nematodes that are attracted to the part of the nematode assay plate or nematodes that have escaped from to the part or (b) measuring an element co-related with the number of the nematodes.

15. The method according to claim 13, wherein the test substance is a test substance that emits an odorant or a test substance that has a flavor.

16. A cancer test method comprising the steps of:

evaluating, by the method according to claim 13, response of nematodes to a biological sample collected from a subject; and
determining, by the step of evaluating, a possibility that the subject has cancer.

17. A method for evaluating response of nematodes to temperature, comprising the steps of:

adjusting a part of a nematode assay plate to a target temperature;
supplying, to a predetermined position, the nematode-encapsulated capsule according to any one of claims 1 to 8;
discharging the nematodes from the nematode-encapsulated capsule; and
after lapse of a predetermined time period, (a) counting the number of nematodes that are attracted to the part of the nematode assay plate or nematodes that have escaped from the part or (b) measuring an element co-related with the number of the nematodes.

18. A nematode response evaluation kit comprising the nematode-encapsulated capsule according to any one of claims 1 to 8.

19. The nematode response evaluation kit according to claim 18, which is for a cancer test.

20. A production kit for producing the nematode-encapsulated capsule according to any one of claims 1 to 4, the production kit comprising the nematode, the water-soluble polymer, and a salt of the cation.

21. A production kit for producing the nematode-encapsulated capsule according to claim 5, the production kit comprising the nematode having been exposed to radiation, the water-soluble polymer, and a salt of the cation.

22. A production kit for producing the nematode-encapsulated capsule according to claim 6, the production kit comprising the nematode having been treated with the hatching inhibitor, the water-soluble polymer, and a salt of the cation.

**23.** A production kit for producing the nematode-encapsulated capsule according to claim 7,
the production kit comprising the nematode having been subjected to a sterilization treatment, the water-soluble polymer, and a salt of the cation.

**24.** A production kit for producing the nematode-encapsulated capsule according to claim 8, wherein,
the production kit comprising the nematode, the water-soluble polymer, and a salt of the cation, and the hatching inhibitor.

FIG. 1

NEMATODE (ADULT)

CAPSULE

1 mm

FIG. 2

1 mm

FIG. 3

1 mm

FIG. 4

NEMATODE EGG

CAPSULE

1 mm

FIG. 5

NEMATODE EGG

CAPSULE  1 mm

IMMEDIATELY AFTER
PRODUCTION OF CAPSULE

AFTER 1 DAY

AFTER 2 DAYS

FIG. 6

NEMATODE (ADULT)

EGG

CAPSULE  1mm

IMMEDIATELY AFTER
PRODUCTION OF CAPSULE

NEMATODE (ADULT)

NEMATODE (LARVA)

AFTER 1 DAY

NEMATODE (ADULT)

NEMATODE (LARVA)

AFTER 2 DAYS

## FIG. 7

CAPSULE

NEMATODE (ADULT)

NEMATODE (LARVA)　1mm

2 DAYS AFTER PRODUCTION OF CAPSULE

## FIG. 8

NEMATODE (ADULT)　　　NEMATODE (ADULT)　　　NEMATODE (ADULT)

EGG　　　　　　　　　　　　EGG　　　　　　　　　　　　EGG

EGG　　　　　　　　EGG　　　　　　　　EGG

CAPSULE　1mm

IMMEDIATELY AFTER　　　AFTER 1 DAY　　　AFTER 2 DAYS
PRODUCTION OF CAPSULE

## FIG. 9

NEMATODE (ADULT)

CAPSULE　1mm

2 DAYS AFTER PRODUCTION OF CAPSULE

EP 4 446 412 A1

FIG. 10

NEMATODE (ADULT)

EGG

CAPSULE           1mm

IMMEDIATELY AFTER
PRODUCTION OF CAPSULE

NEMATODE (ADULT)

AFTER 1 DAY

NEMATODE (ADULT)

AFTER 2 DAYS

FIG. 11

CAPSULE

NEMATODE (ADULT)      1mm

2 DAYS AFTER PRODUCTION OF CAPSULE

FIG. 12

NEMATODE (ADULT)

EGG

CAPSULE                    1mm

IMMEDIATELY AFTER
PRODUCTION OF CAPSULE

NEMATODE (ADULT)

NEMATODE (LARVA)

AFTER 1 DAY

NEMATODE (ADULT)

NEMATODE (LARVA)

AFTER 2 DAYS

FIG. 13

CAPSULE

PLASTIC DISH          1 cm

FIG. 14

NEMATODE CAPSULE

NEMATODE CAPSULE

NEMATODE CAPSULE

LEAF
MOLD

1cm

PLASTIC DISH

IMMEDIATELY AFTER
PRODUCTION OF CAPSULE

AFTER 1 DAY

AFTER 2 DAYS

AFTER 5 DAYS

EP 4 446 412 A1

FIG. 15

NEMATODE

CAPSULE

5 mm

IMMEDIATELY AFTER
SUPPLIED
(LEFT TO STAND)

AFTER 12 MINUTES

FIG. 16

CAPSULE    RECESSED PART

NEMATODE LIQUID

5 mm

IMMEDIATELY AFTER
SUPPLIED
(LEFT TO STAND)

AFTER 3 MINUTES

FIG. 17

NEMATODE

NEMATODE LIQUID  5 mm

IMMEDIATELY AFTER
SUPPLIED
(LEFT TO STAND)

AFTER 3 MINUTES

FIG. 18

RECESSED PART

NEMATODE LIQUID  5 mm

IMMEDIATELY AFTER
SUPPLIED
(LEFT TO STAND)

AFTER 6 MINUTES

FIG. 19

FIG. 20

# FIG. 21

START PRODUCTION OF NEMATODE-
ENCAPSULATED CAPSULE

DETERMINE VARIOUS CONDITIONS SUCH AS
STAGE OF NEMATODE TO BE ENCAPSULATED — S1
IN CAPSULE, AND CULTURE

SELECT WATER-SOLUBLE POLYMER AND CATION AQUEOUS
SOLUTION, DETERMINE VARIOUS CONDITIONS SUCH AS — S2
CONCENTRATION, AND PREPARE

S3
ENCAPSULATE
NEMATODE PRIOR TO
NO ◇ FORMATION OF OUTER PHASE ◇ YES
MEMBRANE OF
CAPSULE?

S6
FORM OUTER PHASE MEMBRANE OF
CAPSULE

S4
INJECT NEMATODE INTO SOLUTION
CONTAINING WATER-SOLUBLE POLYMER

S5
FORM OUTER PHASE MEMBRANE OF
CAPSULE

S7
INJECT NEMATODE                     YES
INTO CAPSULE?

NO

S10
NO ◇ HAS NEMATODE
BEEN ENCAPSULATED IN
CAPSULE?

YES

INJECT NEMATODE INTO CAPSULE — S8

SEAL NEMATODE INJECTION OPENING — S9
OF OUTER PHASE MEMBRANE

S11
SUPERIMPOSE
◇ ANOTHER MEMBRANE FURTHER ◇ YES
ON OUTER PHASE
MEMBRANE?

NO

COMPLETE NEMATODE-ENCAPSULATED
CAPSULE

## INTERNATIONAL SEARCH REPORT

| International application No. |
| --- |
| **PCT/JP2022/045039** |

**A. CLASSIFICATION OF SUBJECT MATTER**

*C12N 11/04*(2006.01)i; *C12Q 1/02*(2006.01)i; *G01N 33/48*(2006.01)i
FI:   C12N11/04; G01N33/48 Z; C12Q1/02

According to International Patent Classification (IPC) or to both national classification and IPC

**B. FIELDS SEARCHED**

Minimum documentation searched (classification system followed by classification symbols)

C12N11/04; C12Q1/02; G01N33/48

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | KIM, J. et al. Calcium alginate beads as a formulation for the application of entomopathogenic nematodes to control rootworms. J Pest Sci. 26 February 2021, vol. 94, pp. 1197-1208 abstract, materials and methods, fig. 1 | 1, 2, 4, 6-10, 20, 24 |
| A | WO 2020/218501 A1 (NATIONAL INSTITUTES FOR QUANTUM AND RADIOLOGICALSCIENCE AND TECHNOLOGY) 29 October 2020 (2020-10-29) entire text | 1-24 |
| A | BURNETT, K. et al. Rapid and gentle hydrogel encapsulation of living organisms enables long-term microscopy over multiple hours. Commun Biol. 2018, vol. 1, no. 73, pp. 1-10 results, fig. 1 | 1-24 |
| A | JP 2021-26004 A (UNIV KUMAMOTO) 22 February 2021 (2021-02-22) entire text | 1-24 |
| A | WO 2019/225633 A1 (NATIONAL INSTITUTES FOR QUANTUM AND RADIOLOGICALSCIENCE AND TECHNOLOGY) 28 November 2019 (2019-11-28) entire text | 1-24 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **07 February 2023** | **21 February 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/045039**

| Patent document cited in search report | | | Publication date (day/month/year) | Patent family member(s) | | | Publication date (day/month/year) |
|---|---|---|---|---|---|---|---|
| WO | 2020/218501 | A1 | 29 October 2020 | US | 2022/0187274 | A1 | |
| | | | | whole document | | | |
| JP | 2021-26004 | A | 22 February 2021 | (Family: none) | | | |
| WO | 2019/225633 | A1 | 28 November 2019 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018061515 A
- WO 2020218501 A
- JP 7133209 A
- JP 5092909 A

**Non-patent literature cited in the description**

- **STIERNAGLE, T.** Maintenance of C. elegans. Worm-Book, 11 February 2006
- **AKIRA HIGASHIBATA et al.** Microgravity elicits reproducible alterations in cytoskeletal and metabolic gene and protein expression in space-flown Caenorhabditis elegans. *npj Microgravity,* 2016, vol. 2, 15022